# EUROPEAN PATENT SPECIFICATION

(11) **EP 2 023 926 B1**
(45) Date of publication and mention of the grant of the patent: **12.10.2011**
(21) Application number: 07861312.2
(22) Date of filing: 18.05.2007
(51) Int. Cl.: A61K 31/44

(54) **PYRIDONECARBOXAMIDE DERIVATIVES USEFUL IN TREATING HYPER-PROLIFERATIVE AND ANGIOGENESIS DISORDERS**
PYRIDONCARBOXAMIDDERIVATE ZUR BEHANDLUNG HYPERPROLIFERATIVER UND ANGIOGENESE-VERMITTELTER LEIDEN
DÉRIVÉS DE PYRIDONECARBOXAMIDE UTILES POUR TRAITER DES TROUBLES HYPERPROLIFÉRATIFS ET LIÉS À L'ANGIOGENÈSE

(30) Priority: 19.05.2006 US 801700 P
(43) Date of publication of application: 18.02.2009
(73) Proprietor: Bayer Pharma Aktiengesellschaft, 13353 Berlin (DE)
(72) Inventor: BOYER, Stephen, Bethany, CT 06524 (US); CANTIN, David, Hamden, CT 06518 (US); LIANG, Sidney X., Bethany, CT 06524 (US)
(86) International application number: PCT/US2007/011981
(87) International publication number: WO 2008/048375

(56) References cited:
- WO-A1-2006/116713
- WO-A2-2006/004636
- US-A1- 2005 239 820
- US-A1- 2005 245 530
- US-A1- 2005 256 123
- US-A1- 2006 004 006
- US-A1- 2006 035 938
- Web page http://www.healthscout.com/ency/68/329/main .html (LIGIBEL) 10 June 2005 XP008104104
- Web page http://www.healthscout.com/ency/68/178/main .html (KNOPF) 22 June 2005 XP008104103

## Description

### BACKGROUND OF THE INVENTION

### 1. Field of the Invention

The present invention relates to novel compounds, pharmaceutical compositions containing such compounds, and the use of those compounds or compositions in treating hyper-proliferative and/or angiogenesis disorders. More particularly, the present invention relates to the use of those compounds or compositions in treating hyper-proliferative and/or angiogenesis disorders as a sole agent or in combination with other active ingredients, such as, in cytotoxic therapies.

### 2. Description of the Related Art

To support progressive tumor growth beyond the size of 1-2 mm³, it is recognized that tumor cells require a functional stroma, a support structure consisting of fibroblast, smooth muscle cells, endothelial cells, extracellular matrix proteins, and soluble factors (Folkman, J., Semin Oncol, 2002, 29(6 Suppl 16), 15-8. Tumors induce the formation of stromal tissues through the secretion of soluble growth factors such as PDGF and transforming growth factor-beta (TGF-beta), which in turn stimulate the secretion of complimentary factors by host cells such as fibroblast growth factor (FGF), epidermal growth factor (EGF), and vascular endothelial growth factor (VEGF). These stimulatory factors induce the formation of new blood vessels, or angiogenesis, which brings oxygen and nutrients to the tumor and allows it to grow and provides a route for metastasis. It is believed some therapies directed at inhibiting stroma formation will inhibit the growth of epithelial tumors from a wide variety of histological types (George, D. Semin Oncol, 2001. 28(5 Suppl 17), 27-33; Shaheen, R.M., et al., Cancer Res, 2001. 61 (4), 1464-8; Shaheen, R.M., et al. Cancer Res, 1999. 59(21), 5412-6). However, because of the complex nature and the multiple growth factors involved in angiogenesis process and tumor progression, an agent targeting a single pathway may have limited efficacy. It is desirable to provide treatment against a number of key signaling pathways utilized by tumors to induce angiogenesis in the host stroma. These include, for example, PDGF, a potent stimulator of stroma formation (Ostman, A. and C.H. Heldin, Adv Cancer Res, 2001, 80, 1-38), FGF, a chemo-attractant and mitogen for fibroblasts and endothelial cells, and VEGF, a potent regulator of vascularization. HGF (hepatocyte growth factor) represents an additional signaling growth factor of interest.

PDGF is a key regulator of stromal formation, which is secreted by many tumors in a paracrine fashion and is believed to promote the growth of fibroblasts, smooth muscle and endothelial cells, promoting stroma formation and angiogenesis. PDGF was originally identified as the v-sis oncogene product of the simian sarcoma virus (Heldin, C.H., et al., J Cell Sci Suppl, 1985, 3, 65-76). The growth factor is made up of two peptide chains, referred to as A or B chains which share 60% homology in their primary amino acid sequence. The chains are disulfide cross linked to form the 30 kDa mature protein composed of either AA, BB or AB homo- or heterodimers. PDGF is found at high levels in platelets, and is expressed by endothelial cells and vascular smooth muscle cells. In addition, the production of PDGF is up regulated under low oxygen conditions such as those found in poorly vascularized tumor tissue (Kourembanas, S., et al., Kidney Int, 1997, 51 (2), 438-43). PDGF binds with high affinity to the PDGF receptor, a 1106 amino acid 124 kDa transmembrane tyrosine kinase receptor (Heldin, C.H., A. Ostman, and L. Ronnstrand, Biochim Biophys Acta, 1998, 1378(1), 79-113). PDGFR is found as homo- or heterodimer chains which have 30% homology overall in their amino acid sequence and 64% homology between their kinase domains (Heldin, C.H., et al. Embo J, 1988, 7(5), 1387-93). PDGFR is a member of a family of tyrosine kinase receptors with split kinase domains that includes VEGFR2 (KDR), VEGFR3 (FIt4), c-Kit, and FLT3. The PDGF receptor is expressed primarily on fibroblast, smooth muscle cells, and pericytes and to a lesser extent on neurons, kidney mesangial, Leydig, and Schwann cells of the central nervous system. Upon binding to the receptor, PDGF induces receptor dimerization and undergoes auto- and transphosphorylation of tyrosine residues which increase the receptors' kinase activity and promotes the recruitment of downstream effectors through the activation of SH2 protein binding domains. A number of signaling molecules form complexes with activated PDGFR including PI-3-kinase, phospholipase C-gamma, src and GAP (GTPase activating protein for p21-ras) (Soskic, V., et al. Biochemistry, 1999, 38(6), 1757-64). Through the activation of PI-3-kinase, PDGF activates the Rho signaling pathway inducing cell motility and migration, and through the activation of GAP, induces mitogenesis through the activation of p21-ras and the MAPK signaling pathway.

In adults, it is believed the major function of PDGF is to facilitate and increase the rate of wound healing and to maintain blood vessel homeostasis (Baker, E.A. and D.J. Leaper, Wound Repair Regen, 2000. 8(5), 392-8; Yu, J., A. Moon, and H.R. Kim, Biochem Biophys Res Commun, 2001, 282(3), 697-700). PDGF is found at high concentrations in platelets and is a potent chemoattractant for fibroblast, smooth muscle cells, neutrophils and macrophages. In addition to its role in wound healing PDGF is known to help maintain vascular homeostasis. During the development of new blood vessels, PDGF recruits pericytes and smooth muscle cells that are needed for the structural integrity of the vessels. PDGF is thought to play a similar role during tumor neovascularization. As part of its role in angiogenesis PDGF controls interstitial fluid pressure, regulating the permeability of vessels through its regulation of the interaction between connective tissue cells and the extracellular matrix. Inhibiting PDGFR activity can lower interstitial pressure and facilitate the influx of cytotoxics into tumors improving the anti-tumor efficacy of these agents (Pietras, K., et al. Cancer Res, 2002, 62(19), 5476-84; Pietras, K., et al. Cancer Res, 2001, 61(7), 2929-34).

PDGF can promote tumor growth through either the paracrine or autocrine stimulation of PDGFR receptors on stromal cells or tumor cells directly, or through the amplification of the receptor or activation of the receptor by recombination. Over expressed PDGF can transform human melanoma cells and keratinocytes (Forsberg, K., et al. Proc Natl Acad Sci USA., 1993, 90(2), 393-7; Skobe, M. and N.E. Fusenig, Proc Natl Acad Sci USA, 1998, 95(3), 1050-5), two cell types that do not express PDGF receptors, presumably by the direct effect of PDGF on stroma formation and induction of angiogenesis. This paracrine stimulation of tumor stroma is also observed in carcinomas of the colon, lung, breast, and prostate (Bhardwaj, B., et al. Clin Cancer Res, 1996, 2(4), 773-82; Nakanishi, K., et al. Mod Pathol, 1997, 10(4), 341-7; Sundberg, C., et al. Am J Pathol, 1997, 151(2), 479-92; Lindmark, G., et al. Lab Invest, 1993, 69(6), 682-9; Vignaud, J.M., et al, Cancer Res, 1994, 54(20), 5455-63) where the tumors express PDGF, but not the receptor.

The autocrine stimulation of tumor cell growth, where a large faction of tumors analyzed express both the ligand PDGF and the receptor, has been reported in glioblastomas (Fleming, T.P., et al. Cancer Res, 1992, 52(16), 4550-3), soft tissue sarcomas (Wang, J., M.D. Coltrera, and A.M. Gown, Cancer Res, 1994, 54(2), 560-4) and cancers of the ovary (Henriksen, R., et al. Cancer Res, 1993, 53(19), 4550-4), prostate (Fudge, K., C.Y. Wang, and M.E. Steams, Mod Pathol, 1994, 7(5), 549-54), pancreas (Funa, K., et al. Cancer Res, 1990, 50(3), 748-53) and lung (Antoniades, H.N., et al., Proc Natl Acad Sci USA, 1992, 89(9), 3942-6). Ligand independent activation of the receptor is found to a lesser extent but has been reported in chronic myelomonocytic leukemia (CMML) where the a chromosomal translocation event forms a fusion protein between the Ets-like transcription factor TEL and the PDGF receptor. In addition, activating mutations in PDGFR have been found in gastrointestinal stromal tumors in which c-Kit activation is not involved (Heinrich, M.C., et al., Science, 2003, 9, 9).

Certain PDGFR inhibitors will interfere with tumor stromal development and are believed to inhibit tumor growth and metastasis.

Another major regulator of angiogenesis and vasculogenesis in both embryonic development and some angiogenic-dependent diseases is vascular endothelial growth factor (VEGF; also called vascular permeability factor, VPF). VEGF represents a family of isoforms of mitogens existing in homodimeric forms due to alternative RNA splicing. The VEGF isoforms are reported to be highly specific for vascular endothelial cells (for reviews, see: Farrara et al. Endocr. Rev. 1992, 13, 18; Neufield et al. FASEB J. 1999, 13, 9).

VEGF expression is reported to be induced by hypoxia (Shweiki et al. Nature 1992, 359, 843), as well as by a variety of cytokines and growth factors, such as interleukin-1, interleukin-6, epidermal growth factor and transforming growth factor. To date, VEGF and the VEGF family members have been reported to bind to one or more of three transmembrane receptor tyrosine kinases (Mustonen et al. J. Cell Biol., 1995, 129, 895), VEGF receptor-1 (also known as flt-1 (fms-like tyrosine kinase-1)), VEGFR-2 (also known as kinase insert domain containing receptor (KDR); the murine analogue of KDR is known as fetal liver kinase-1 (flk-1)), and VEGFR-3 (also known as flt-4). KDR and flt-1 have been shown to have different signal transduction properties (Waltenberger et al. J. Biol. Chem. 1994, 269, 26988); Park et al. Oncogene 1995, 10, 135). Thus, KDR undergoes strong ligand-dependant tyrosine phosphorylation in intact cells, whereas flt-1 displays a weak response. Thus, binding to KDR is believed to be a critical requirement for induction of the full spectrum of VEGF-mediated biological responses.

*In vivo*, VEGF plays a central role in vasculogenesis, and induces angiogenesis and permeabilization of blood vessels. Deregulated VEGF expression contributes to the development of a number of diseases that are characterized by abnormal angiogenesis and/or hyperpermeability processes. It is believed regulation of the VEGF-mediated signal transduction cascade by some agents can provide a useful mode for control of abnormal angiogenesis and/or hyperpermeability processes.

The vascular endothelial growth factors (VEGF, VEGF-C, VEGF-D) and their receptors (VEGFR2, VEGFR3) are not only key regulators of tumor angiogenesis, but also lymphangiogenesis. VEGF, VEGF-C and VEGF-D are expressed in most tumors, primarily during periods of tumor growth and, often at substantially increased levels. VEGF expression is stimulated by hypoxia, cytokines, oncogenes such as *ras,* or by inactivation of tumor suppressor genes (McMahon, G. Oncologist 2000, 5(Suppl. 1), 3-10; McDonald, N.Q.; Hendrickson, W.A. Cell 1993, 73, 421-424).

The biological activities of the VEGFs are mediated through binding to their receptors. It is believed VEGFR3 (also called Flt-4) is predominantly expressed on lymphatic endothelium in normal adult tissues and that VEGFR3 function is needed for new lymphatic vessel formation, but not for maintenance of the pre-existing lymphatics. VEGFR3 is also upregulated on blood vessel endothelium in tumors. Recently VEGF-C and VEGF-D, ligands for VEGFR3, have been identified as regulators of lymphangiogenesis in mammals. Lymphangiogenesis induced by tumor-associated lymphangiogenic factors could promote the growth of new vessels into the tumor, providing tumor cells access to systemic circulation. Cells that invade the lymphatics could find their way into the bloodstream via the thoracic duct. Tumor expression studies have allowed a direct comparison of VEGF-C, VEGF-D and VEGFR3 expression with clinicopathological factors that relate directly to the ability of primary tumors to spread (e.g., lymph node involvement, lymphatic invasion, secondary metastases, and disease-free survival). In many instances, these studies demonstrate a statistical correlation between the expression of lymphangiogenic factors and the ability of a primary solid tumor to metastasize (Skobe, M. et al. Nature Med. 2001, 7(2), 192-198; Stacker, S.A. et al. Nature Med. 2001, 7(2), 186-191; Makinen, T. et al. Nature Med. 2001, 7(2), 199-205; Mandriota, S.J. et al. EMBO J. 2001, 20(4), 672-82; Karpanen, T. et al. Cancer Res. 2001, 61(5), 1786-90; Kubo, H. et al. Blood 2000, 96(2), 546-53).

Hypoxia appears to be an important stimulus for VEGF production in malignant cells. Activation of p38 MAP kinase is required for VEGF induction by tumor cells in response to hypoxia (Blaschke, F. et al. Biochem. Biophys. Res. Commun. 2002, 296, 890-896; Shemirani, B. et al. Oral Oncology 2002, 38, 251-257). In addition to its involvement in angiogenesis through regulation of VEGF secretion, p38 MAP kinase promotes malignant cell invasion, and migration of different tumor types through regulation of collagenase activity and urokinase plasminogen activator expression (Laferriere, J. et al. J. Biol. Chem. 2001, 276, 33762-33772; Westermarck, J. et al. Cancer Res. 2000, 60, 7156-7162; Huang, S. et al. J. Biol. Chem. 2000, 275, 12266-12272; Simon, C. et al. Exp. Cell Res. 2001, 271, 344-355).

The proto-oncogene c-Met, a member of the receptor tyrosine kinase family, encodes a heterodimeric complex consisting of a 140-kDa membrane-spanning β chain and a 50-kDa extracellular α chain. This heterodimeric complex acts as a high-affinity receptor for hepatocyte growth factor (HGF) or scatter factor (SF). c-Met/HGF signaling is required for normal mammalian development and has been shown to be particularly important in cell growth, migration, morphogenic differentiation, and organization of three-dimensional tubular structures (e.g. renal tubular cells, gland formation, etc.). c-Met and HGF are widely expressed in a variety of tissues, and their expression is normally confined to cells of epithelial and mesenchymal origin, respectively. There are now several lines of compelling evidence that HGF/c-Met signaling has an important role in the development and malignant progression of tumors of various histological types. Cell lines that ectopically overexpress c-Met or HGF become tumorigenic and metastatic in nude mice, whereas c-Met downregulation decreases their tumorigenic potential. HGF-dependent autocrine loops are found associated with osteosarcomas, rhabdomyosarcomas and breast carcinomas (Trusolino and Comoglio, Nat Rev Cancer, 2002, 2, 289-300). c-Met or HGF transgenic mice develop metastatic tumors (Wang, R. et al., J. Cell Biol. 2001, 153, 1023-1034; Takayama et al., Proc. Natl. Acad. Sci. U.S.A. 1997, 94, 701-706). Over-expression of c-Met expression has been found in many kinds of solid tumors and correlates with poor prognosis (Birchmeier, et al. Mol. Cell Biol., 2003, 4, 915-925; Christensen, J. and Salgia, R., Can Lett., 2005, 225, 1-26). The unequivocal evidence linking c-Met and human cancer comes from the identification of germline activating mutations in patients suffering from hereditary papillary renal carcinomas (Dharmawardana, et al., Curr. Mol. Med., 2004, 4, 855-868). Finally, amplification of the c-Met gene was observed in many gastric tumors (Ponzetto, C. et al., Oncogene. 1991, 6, 553-9).

Due to a strong link between c-Met/HGF signaling pathway and tumorigenesis and tumor progression, several therapeutic approaches have been taken by various groups. HGF/SF-neutralizing antibodies (Cao et al., Proc Natl Acad Sci USA 2001, 98, 7443-8), c-Met antisense oligonucleotides (Kitamura et al., Br J Cancer 2000, 83: 668-73), dominant-negative forms of the Met protein (Firon et al., Oncogene 2000, 19, 2386-97; Furge et al., Proc Natl Acad Sci USA 2001, 98, 10722-7), ribozymes that target Met mRNA (Abounader et al., J Natl Cancer Inst, 1999, 91, 1548-56; Abounader et al., FASEB J 2002, 16, 108 -10), and small molecule c-Met tyrosine kinase inhibitor (Christensen et al., Cancer Res 2003, 63, 7345-55) are being investigated as possible strategies to block c-Met activation and suppress tumor growth, invasion, and metastasis. Identification of a potent inhibitor of c-Met kinase activity therefore has the great potential to inhibit tumor growth of various cancers types.

Chronic myelogenous leukemia (CML) is caused by the oncogenic protein, Bcr-Abl (Groffen, J. et al., J Cell Physiol Suppl, 1984, 3, 179-191, Sattler, M. and Griffin, J. D., Semin Hematol, 2003, 40, 4-10). The Philadelphia chromosome, which is the hallmark of CML, is formed in CML patients due to a reciprocal translocation between chromosomes 9 and 22 (Rowley, J. D., Nature, 1973, 243, 290-293), and this translocation results in the formation of Bcr-Abl fusion protein (Groffen, J. and Heisterkamp, N., Baillieres Clin Haematol, 1987, 1, 983-999). Abl protein is a non-receptor tyrosine kinase whose activity is tightly regulated in the normal cells. However, the fusion protein is constitutively activated due to the presence of Bcr protein at the N-terminus. The constitutively active protein transforms at the myeloid the blast cell stage thus giving rise to CML (Kelliher, M. A., et al., Proc Natl Acad Sci USA, 1990, 87, 6649-6653). Depending on the exact breakpoints at the chromosomes involved in the translocation, the size of the fusion protein varies from 185 to 230 kDa, although 210 kDa protein being the most commoon in CML.

Development of Imanitib as an inhibitor of Bcr-Abl protein to treat the CML pateints has pioneered the field of targeted therapy in oncology (Capdeville, R., et al., Nat Rev Drug Discov, 2002, 1, 493-502). Patients with early phase CML were found to respond to a degree of greater than 90% at both haematological and cytogenetic levels (Deininger, M. et al., Blood, 2005, 105, 2640-2653, Talpaz, M. et al., Blood, 2002, 99, 1928-1937). However, most patients after a prolonged treatment develop resistance to Imanitib (Gorre, M. E. and Sawyers, C. L., Curr Opin Hematol, 2002, 9, 303-307). To date, more than 30 Imanitib resitant mutations have been observed in patients and most of these mutations are confined to a sub-domain within the kinase region of the fusion protein. Importantly, three mutations namely T315I, E255K and M351T represent more than 50% of the Imanitib resistance (Deininger, M., Buchdunger, E. and Druker, B. J., Blood, 2005, 105, 2640-2653).

Recently, there has been much effort in the field of oncology to overcome the Imanitib resistance in CML patients. For example, BMS-354825 has been reported to be an inhibitor of Bcr-Abl and also Src family kinases. Among the 15 Imanitib-resistance mutations tested in cell based assays, BMS-354825 was reported to inhibit all the mutant forms of the protein except T315I (Shah, N. P., et al., Science, 2004, 305, 399-401). The compound AMN-107 has been reported to inhibit Bcr-AbI kinase activity with 20-fold greater potency than Imatinib. AMN-107 was reported to inhib most Imanitib resistant mutations except for T315I. AMN-107 also shows weaker inhibition in a biochemical assay against E255K mutant (IC₅₀ of 400 nM) (Weisberg, E., et al., Cancer Cell, 2005, 7, 129-141). There fore, there is a significant unmet medical need for new therapeutics to treat CML and Imatinib-resistant CML.

Despite the advancements in the art, there remains a need for cancer treatments and anti-cancer compounds. The utility of the compounds of the present invention can be illustrated, for example, by their activity *in vitro* in the assays described below.

### SUMMARY OF THE INVENTION

The present invention provides compounds represented by the formula: 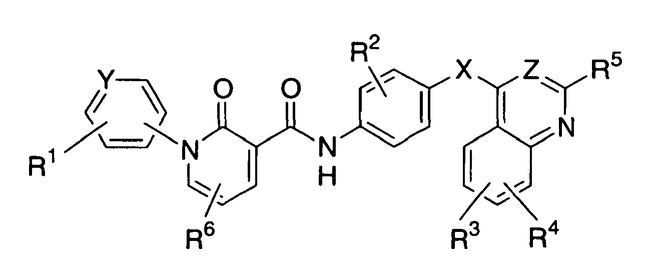 wherein:
X is selected from: O and S;
Y and Z are independently selected from: CH and N;
R¹ is selected from: hydrogen, one or more halogen, NR⁷C(O)R⁸, C(O)OR⁹, OC(O)R¹⁰ OR¹¹, SR¹² cyano, C(O)NR¹⁵R¹⁶, SO₂NR¹⁵R¹⁶, NR¹⁵R¹⁶, linear, branched or cyclic C1-6 alkyl optionally substituted with one or more halogen, OR¹¹, NR¹⁵R¹⁶, - (CH₂)ᵣNR¹⁵R¹⁶ wherein r is 1, 2 or 3, -O-(CH₂)ₚNR¹⁵R¹⁶, and -NR¹⁵-(CH₂)pNR¹⁵R¹⁶ wherein p is 2, 3, or 4;
R² is selected from: hydrogen, one or more halogen, OR¹¹, NR¹⁵R¹⁶ and linear, branched or cyclic C1-6 alkyl optionally substituted with halogen, OR¹¹ or NR¹⁵R¹⁶;
R³ and R⁴ are each independently selected from: hydrogen, one or more halogen, NR⁷C(O)R⁸, C(O)OR⁹, OC(O)R¹⁰, OR¹¹, cyano, C(O)NR¹⁵R¹⁶, SO₂NR¹⁵R¹⁶, NR¹⁵R¹⁶, linear, branched or cyclic C1-6 alkyl optionally substituted with halogen, OR¹¹, NR¹⁵R¹⁶, -(CH₂)ᵣNR¹⁵R¹⁶ wherein r is 1, 2 or 3, -O-(CH₂)ₚNR¹⁵R¹⁵ and -NR¹³-(CH₂)ₚNR¹⁵R¹⁵ wherein p is 2, 3 or 4;
R⁵ is selected from: hydrogen, OR¹¹, NR¹⁵R¹⁶, linear, branched or cyclic C1-6 alkyl optionally substituted with one or more halogen, and -(CH₂)ᵣNR¹⁵R¹⁶ wherein r is 1, 2 or 3;
R⁶ is selected from: hydrogen, NR⁷C(O)R⁸, C(O)OR⁹, OC(O)R¹⁰, OR¹¹, SR¹², C(O)NR¹⁵R¹⁶, SO₂NR¹⁵R²¹, NR¹⁵R¹⁶, linear, branched or cyclic C1-6 alkyl optionally substituted with one or more halogen, OR⁸, NR¹⁵R¹⁶, -(CH₂)ᵣNR¹⁵R¹⁶ wherein r is 1, 2 or 3, -O-(CH₂)ₚNR¹⁵R¹⁶ and
-NR¹³-(CH₂)ₚNR¹⁵R¹⁶ wherein p is 2, 3 or 4;
R⁷ to R¹⁴ are each independently selected from: hydrogen, linear, branched or cyclic C1-6 alkyl optionally substituted with one or more halogen, OR¹¹, and NR¹⁵R¹⁶;
R¹⁵ and R¹⁶ are each independently selected from: hydrogen, linear, branched or cyclic C1-6 alkyl optionally substituted with one or more halogen, OR¹¹, NR¹⁷R¹⁸ and a group wherein R¹⁵ and R¹⁶ together form a five- or six-membered ring optionally containing O or NR¹⁴; and
R¹⁷ and R¹⁸ are each independently selected from: hydrogen, linear, branched or cyclic C1-6 alkyl optionally substituted with one or more halogen, and OR¹¹;
pharmaceutically acceptable salts thereof, metabolites thereof, solvates thereof, hydrates thereof, prodrugs thereof, polymorphs thereof and diastereoisomeric forms thereof including isolated stereoisomers thereof and those within a mixture of stereoisomers.

The present invention also provides a pharmaceutical composition including one or more of the above compounds and a physiologically acceptable carrier.

Also described is a method of treating hyper-proliferative disorders including administering to a mammal in need of treatment a therapeutically effective amount of one or more compounds according to the present invention.

Also described is a method of treating angiogenesis disorders including administering to a mammal in need of therapy a therapeutically effective amount of one or more compounds according to the present invention.

### DESCRIPTION OF THE PREFERRED EMBODIMENTS

The present invention provides:
(i) novel compounds of the present invention include compounds of examples 1-7 represented by the formula: 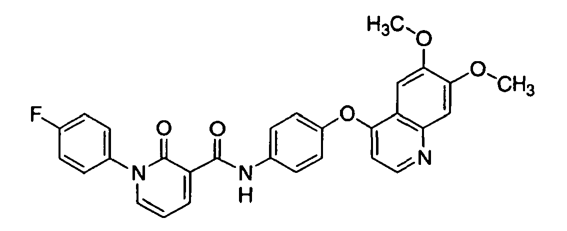 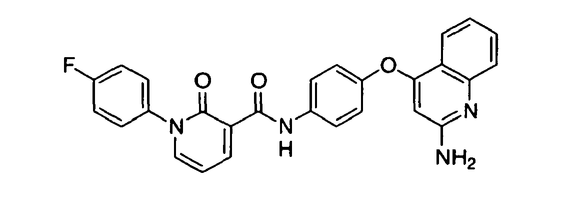 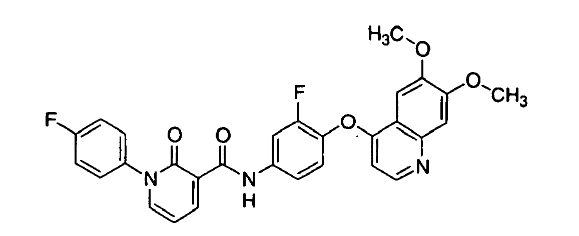 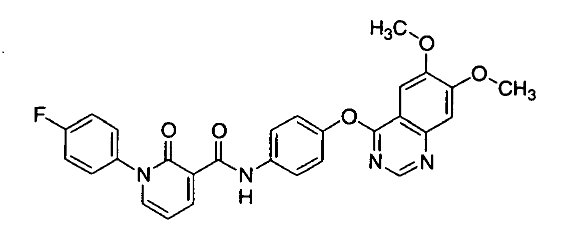 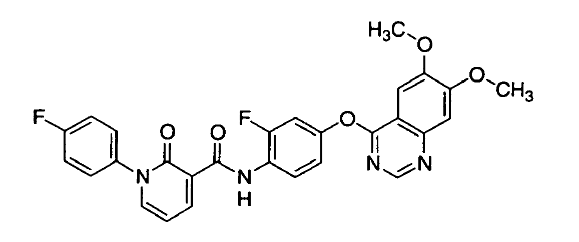 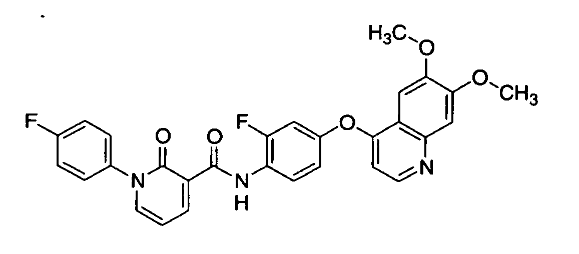 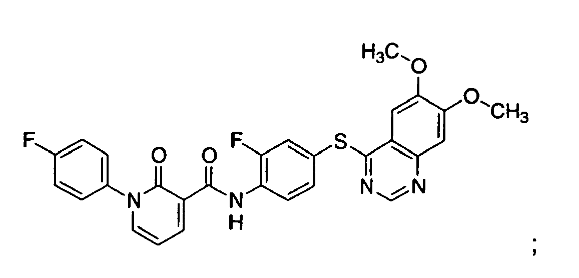
(ii) pharmaceutical compositions containing compounds of examples 1-7 below or pharmaceutically acceptable salts, solvates, hydrates,
   polymorphs and diastereoisomeric forms thereof (both isolated stereoisomers and mixtures of stereoisomers), and also including combinations thereof; and
(iii) use of those compounds of (i) or compositions of (ii) for treating diseases, e.g., hyper-proliferative and/or angiogenesis disorders, as a sole agent or in combination with other active ingredients, e.g., cytotoxic therapies.

The compounds of examples 1-7, salts, solvates, hydrates and thereof, including polymorphs and diastereoisomeric forms (both isolated stereoisomers and mixtures of stereoisomers) and combinations thereof, are collectively referred to herein as the "compounds of the invention."

Where the plural form of the word compounds, salts, polymorphs, hydrates, solvates and the like, is used herein, this is taken to mean also a single compound, salt, polymorph, isomer, hydrate, solvate or the like.

The compounds of this invention may contain one or more asymmetric centers, depending upon the location and nature of the various substituents desired. Asymmetric carbon atoms may be present in the (*R*) or (*S*) configuration or (*R,S*) configuration. In certain instances, asymmetry may also be present due to restricted rotation about a given bond, for example, the central bond adjoining two substituted aromatic rings of the specified compounds. Substituents on a ring may also be present in either cis or trans form. It is intended that all such configurations (including enantiomers and diastereomers), are included within the scope of the present invention. Preferred compounds are those which produce the more desirable biological activity. Separated, pure or partially purified isomers or racemic mixtures of the compounds of this invention are also included within the scope of the present invention. The purification of said isomers and the separation of said isomeric mixtures can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallization. The optically active bases or acids are then liberated from the separated diastereomeric salts. A different process for separation of optical isomers involves the use of chiral chromatography (e.g., chiral HPLC columns), with or without conventional derivation, optimally chosen to maximize the separation of the enantiomers. Suitable chiral HPLC columns are manufactured by Diacel, e.g., Chiracel OD and Chiracel OJ among many others, all routinely selectable. Enzymatic separations, with or without derivitization, are also useful. The optically active compounds of this invention can likewise be obtained by chiral syntheses utilizing optically active starting materials.

The present invention also relates to useful forms of the compounds as disclosed herein, such as pharmaceutically acceptable salts, co-precipitates, metabolites, hydrates, solvates and prodrugs of all the compounds of examples 1-82. The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19. Pharmaceutically acceptable salts include those obtained by reacting the main compound, functioning as a base, with an inorganic or organic acid to form a salt, for example, salts of hydrochloric acid, sulfuric acid, phosphoric acid, methane sulfonic acid, camphor sulfonic acid, oxalic acid, maleic acid, succinic acid and citric acid.

Pharmaceutically acceptable salts also include those in which the main compound functions as an acid and is reacted with an appropriate base to form, e.g., sodium, potassium, calcium, magnesium, ammonium, and chorine salts. Those skilled in the art will further recognize that acid addition salts of the claimed compounds may be prepared by reaction of the compounds with the appropriate inorganic or organic acid via any of a number of known methods. Alternatively, alkali and alkaline earth metal salts are prepared by reacting the compounds of the invention with the appropriate base via a variety of known methods.

Representative salts of the compounds of this invention include the conventional non-toxic salts and the quaternary ammonium salts which are formed, for example, from inorganic or organic acids or bases by means well known in the art. For example, such acid addition salts include acetate, adipate, alginate, ascorbate, aspartate, benzoate, benzenesulfonate, bisulfate, butyrate, citrate, camphorate, camphorsulfonate, cinnamate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, itaconate, lactate, maleate, mandelate, methanesulfonate, 2-naphthalenesulfonate, nicotinate, nitrate, oxalate, pamoate, pectinate, persulfate, 3-phenylpropionate, picrate, pivalate, propionate, succinate, sulfonate, tartrate, thiocyanate, tosylate, and undecanoate.

Base salts include alkali metal salts such as potassium and sodium salts, alkaline earth metal salts such as calcium and magnesium salts, and ammonium salts with organic bases such as dicyclohexylamine and N-methyl-D-glucamine. Additionally, basic nitrogen containing groups may be quatemized with such agents as lower alkyl halides such as methyl, ethyl, propyl, and butyl chlorides, bromides and iodides; dialkyl sulfates like dimethyl, diethyl, and dibutyl sulfate; and diamyl sulfates, long chain halides such as decyl, lauryl, myristyl and strearyl chlorides, bromides and iodides, aralkyl halides like benzyl and phenethyl bromides and others.

Certain compounds of this invention can be further modified with labile functional groups that are cleaved after *in vivo* administration to furnish the parent active agent and the pharmacologically inactive derivatizing (functional) group. These derivatives, commonly referred to as prodrugs, can be used, for example, to alter the physicochemical properties of the active agent, to target the active agent to a specific tissue, to alter the pharmacokinetic and pharmacodynamic properties of the active agent, and to reduce undesirable side effects.

Prodrugs of the compounds of the invention include, e.g., the esters of appropriate compounds of this invention, are well-tolerated, pharmaceutically acceptable esters such as alkyl esters including methyl, ethyl, propyl, isopropyl, butyl, isobutyl or pentyl esters. Additional esters such as phenyl(C₁-C₆)alkyl may be used, although methyl ester is preferred.

Solvates for the purpose of this invention are those forms of the compounds where solvent molecules form a complex in the solid state and include, but are not limited to for example ethanol and methanol. Hydrates are a specific form of solvates where the solvent is water.

Methods for synthesizing prodrugs are described in the following reviews on the subject :
Higuchi, T.; Stella, V., eds., Prodrugs As Novel Drug Delivery Systems. ACS Symposium Series. American Chemical Society: Washington, DC (1975).
Roche, E. B. Design of Biopharmaceutical Properties through Prodrugs and Analogs. American Pharmaceutical Association: Washington, DC (1977).
Sinkula, A. A.; Yalkowsky, S. H. J Pharm Sci. 1975, 64, 181-210.
Stella, V. J.; Charman, W. N. Naringrekar, V. H. Drugs 1985, 29, 455-473.
Bundgaard, H., ed. Design of Prodrugs. Elsevier: New York (1985).
Stella, V. J.; Himmelstein, K. J. J. Med Chem. 1980, 23, 1275-1282.
Han, H-K; Amidon, G. L. AAPS Pharmsci 2000, 2, 1- 11.
Denny, W. A. Eur. J. Med. Chem. 2001, 36, 577-595.
Wermuth, C. G. in Wermuth, C. G., ed., The Practice of Medicinal Chemistry Academic Press: San Diego (1996), 697-715.
Balant, L. P.; Doelker, E. in Wolff, M. E. ed. Burgers Medicinal Chemistry And Drug Discovery John Wiley & Sons: New York (1997), 949-982.

The particular process to be utilized in the preparation of the compounds used in this embodiment of the invention depends upon the specific compound desired. Such factors as the selection of the specific substituents play a role in the path to be followed in the preparation of the specific compounds of this invention. Those factors are readily recognized by one of ordinary skill in the art.

Commonly used pharmaceutical ingredients that can be used as appropriate to formulate the composition for its intended route of administration include:
**acidifying agents** (examples include but are not limited to acetic acid, citric acid, fumaric acid, hydrochloric acid, nitric acid);
**alkalinizing agents** (examples include but are not limited to ammonia solution, ammonium carbonate, diethanolamine, monoethanolamine, potassium hydroxide, sodium borate, sodium carbonate, sodium hydroxide, triethanolamine, trolamine);
**adsorbents** (examples include but are not limited to powdered cellulose and activated charcoal);
**aerosol propellants** (examples include but are not limited to carbon dioxide, CCl₂F₂, F₂ClC-CClF₂ and CClF₃)
**air displacement agents** (examples include but are not limited to nitrogen and argon);
**antifungal preservatives** (examples include but are not limited to benzoic acid, butylparaben, ethylparaben, methylparaben, propylparaben, sodium benzoate);
**antimicrobial preservatives** (examples include but are not limited to benzalkonium chloride, benzethonium chloride, benzyl alcohol, cetylpyridinium chloride, chlorobutanol, phenol, phenylethyl alcohol, phenylmercuric nitrate and thimerosal);
**antioxidants** (examples include but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite);
**binding materials** (examples include but are not limited to block polymers, natural and synthetic rubber, polyacrylates, polyurethanes, silicones, polysiloxanes and styrene-butadiene copolymers);
**buffering agents** (examples include but are not limited to potassium metaphosphate, dipotassium phosphate, sodium acetate, sodium citrate anhydrous and sodium citrate dihydrate);
**carrying agents** (examples include but are not limited to acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection);
**chelating agents** (examples include but are not limited to edetate disodium and edetic acid);
**colorants** (examples include but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red);
**clarifying agents** (examples include but are not limited to bentonite);
**emulsifying agents** (examples include but are not limited to acacia, cetomacrogol, cetyl alcohol, glyceryl monostearate, lecithin, sorbitan monooleate, polyoxyethylene 50 monostearate);
**encapsulating agents** (examples include but are not limited to gelatin and cellulose acetate phthalate);
**flavorants** (examples include but are not limited to anise oil, cinnamon oil, cocoa, menthol, orange oil, peppermint oil and vanillin);
**humectants** (examples include but are not limited to glycerol, propylene glycol and sorbitol);
**levigating agents** (examples include but are not limited to mineral oil and glycerin);
**oils** (examples include but are not limited to arachis oil, mineral oil, olive oil, peanut oil, sesame oil and vegetable oil);
**ointment bases** (examples include but are not limited to lanolin, hydrophilic ointment, polyethylene glycol ointment, petrolatum, hydrophilic petrolatum, white ointment, yellow ointment, and rose water ointment);
**penetration enhancers (transdermal delivery)** (examples include but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas)
**plasticizers** (examples include but are not limited to diethyl phthalate and glycerol);
**solvents** (examples include but are not limited to ethanol, corn oil, cottonseed oil, glycerol, isopropanol, mineral oil, oleic acid, peanut oil, purified water, water for injection, sterile water for injection and sterile water for irrigation);
**stiffening agents** (examples include but are not limited to cetyl alcohol, cetyl esters wax, microcrystalline wax, paraffin, stearyl alcohol, white wax and yellow wax);
**suppository bases** (examples include but are not limited to cocoa butter and polyethylene glycols (mixtures));
**surfactants** (examples include but are not limited to benzalkonium chloride, nonoxynol 10, oxtoxynol 9, polysorbate 80, sodium lauryl sulfate and sorbitan monopalmitate);
**suspending agents** (examples include but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum);
**sweetening agents** (examples include but are not limited to aspartame, dextrose, glycerol, mannitol, propylene glycol, saccharin sodium, sorbitol and sucrose);
**tablet anti-adherents** (examples include but are not limited to magnesium stearate and talc);
**tablet binders** (examples include but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch);
**tablet and capsule diluents** (examples include but are not limited to dibasic calcium phosphate, kaolin, lactose, mannitol, microcrystalline cellulose, powdered cellulose, precipitated calcium carbonate, sodium carbonate, sodium phosphate, sorbitol and starch);
**tablet coating agents** (examples include but are not limited to liquid glucose, hydroxyethyl cellulose, hydroxypropyl cellulose, hydroxypropyl methylcellulose, methylcellulose, ethylcellulose, cellulose acetate phthalate and shellac);
**tablet direct compression excipients** (examples include but are not limited to dibasic calcium phosphate);
**tablet disintegrants** (examples include but are not limited to alginic acid, carboxymethylcellulose calcium, microcrystalline cellulose, polacrillin potassium, cross-linked polyvinylpyrrolidone, sodium alginate, sodium starch glycollate and starch);
**tablet glidants** (examples include but are not limited to colloidal silica, corn starch and talc);
**tablet lubricants** (examples include but are not limited to calcium stearate, magnesium stearate, mineral oil, stearic acid and zinc stearate);
**tablet/capsule opaquants** (examples include but are not limited to titanium dioxide);
**tablet polishing agents** (examples include but are not limited to camuba wax and white wax);
**thickening agents** (examples include but are not limited to beeswax, cetyl alcohol and paraffin);
**tonicity agents** (examples include but are not limited to dextrose and sodium chloride);
**viscosity increasing agents** (examples include but are not limited to alginic acid, bentonite, carbomers, carboxymethylcellulose sodium, methylcellulose, polyvinyl pyrrolidone, sodium alginate and tragacanth); and
**wetting agents** (examples include but are not limited to heptadecaethylene oxycetanol, lecithins, sorbitol monooleate, polyoxyethylene sorbitol monooleate, and polyoxyethylene stearate).

Pharmaceutical compositions according to the present invention can be illustrated as follows:

### Sterile Solution

A 5 mg/mL solution of the desired compound of this invention can be made using sterile, injectable water, and the pH is adjusted if necessary. The solution is diluted for administration to 1 - 2 mg/mL with sterile 5% dextrose and is administered as an IV infusion over about 60 minutes.

### Lyophilized powder for administration

A sterile preparation can be prepared with (i) 100 - 1000 mg of the desired compound of this invention as a lypholized powder, (ii) 32- 327 mg/mL sodium citrate, and (iii) 300 - 3000 mg Dextran 40. The formulation is reconstituted with sterile, injectable saline or dextrose 5% to a concentration of 10 to 20 mg/mL, which is further diluted with saline or dextrose 5% to 0.2 - 0.4 mg/mL, and is administered either IV bolus or by IV infusion over 15 - 60 minutes.

### Intramuscular suspension

The following solution or suspension can be prepared, for intramuscular injection:
50 mg/mL of the desired, water-insoluble compound of this invention
5 mg/mL sodium carboxymethylcellulose
4 mg/mL TWEEN 80
9 mg/mL sodium chloride
9 mg/mL benzyl alcohol

### Hard Shell Capsules

A large number of unit capsules are prepared by filling standard two-piece hard galantine capsules each with 100 mg of powdered active ingredient, 150 mg of lactose, 50 mg of cellulose and 6 mg of magnesium stearate.

### Soft Gelatin Capsules

A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.

### Tablets

A large number of tablets are prepared by conventional procedures so that the dosage unit is 100 mg of active ingredient, 0.2 mg. of colloidal silicon dioxide, 5 mg of magnesium stearate, 275 mg of microcrystalline cellulose, 11 mg. of starch, and 98.8 mg of lactose. Appropriate aqueous and non-aqueous coatings may be applied to increase palatability, improve elegance and stability or delay absorption.

### Immediate Release Tablets/Capsules

These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid state extraction techniques. The drug compounds may be compressed with viscoelastic and thermoelastic sugars and polymers or effervescent components to produce porous matrices intended for immediate release, without the need of water.

The present invention also provides a method of treating hyper-proliferative Disorders. The method includes the step of administering to a mammal in need of treatment a therapeutically effective amount of a pharmaceutical compositon according to the present invention.

Also described is a method of treating angiogenesis disorders including administering to a mammal in need of therapy a therapeutically effective amount of one or more compounds according to the present invention described herein below.

### Method of treating hyper-proliferative disorders

The present invention relates to a method for using the compounds of the present invention and compositions thereof, to treat mammalian hyper-proliferative disorders. Compounds can be utilized to inhibit, block, reduce, decrease, etc., cell proliferation and/or cell division, and/or produce apoptosis. This method includes administering to a mammal in need thereof, including a human, an amount of a compound of this invention, (Compounds of examples 1-7 or a pharmaceutically acceptable salt, isomer, polymorph, metabolite, hydrate, solvate or ester thereof; etc.) which is effective to treat the disorder. Hyper-proliferative disorders include but are not limited, e.g., psoriasis, keloids, and other hyperplasias affecting the skin, benign prostate hyperplasia (BPH), solid tumors, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukemias.

Examples of breast cancer include, but are not limited to invasive ductal carcinoma, invasive lobular carcinoma, ductal carcinoma in situ, and lobular carcinoma in situ.

Examples of cancers of the respiratory tract include, but are not limited to small-cell and non-small-cell lung carcinoma, as well as bronchial adenoma and pleuropulmonary blastoma.

Examples of brain cancers include, but are not limited to brain stem and hypophtalmic glioma, cerebellar and cerebral astrocytoma, medulloblastoma, ependymoma, as well as neuroectodermal and pineal tumor.

Tumors of the male reproductive organs include, but are not limited to prostate and testicular cancer. Tumors of the female reproductive organs include, but are not limited to endometrial, cervical, ovarian, vaginal, and vulvar cancer, as well as sarcoma of the uterus.

Tumors of the digestive tract include, but are not limited to anal, colon, colorectal, esophageal, gallbladder, gastric, pancreatic, rectal, small-intestine, and salivary gland cancers.

Tumors of the urinary tract include, but are not limited to bladder, penile, kidney, renal pelvis, ureter, urethral and human papillary renal cancers.

Eye cancers include, but are not limited to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to squamous cell carcinoma, Kaposi's sarcoma, malignant melanoma, Merkel cell skin cancer, and non-melanoma skin cancer.

Head-and-neck cancers include, but are not limited to laryngeal, hypopharyngeal, nasopharyngeal, oropharyngeal cancer, lip and oral cavity cancer and squamous cell. Lymphomas include, but are not limited to AIDS-related lymphoma, non-Hodgkin's lymphoma, cutaneous T-cell lymphoma, Burkitt lymphoma, Hodgkin's disease, and lymphoma of the central nervous system. Sarcomas include, but are not limited to sarcoma of the soft tissue, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

These disorders have been well characterized in humans, but also exist with a similar etiology in other mammals, and can be treated by administering pharmaceutical compositions of the present invention.

The term "treating" or "treatment" as stated throughout this discussed is used conventionally, e.g., the management or care of a subject for the purpose of combating, alleviating, reducing, relieving, improving the condition of, etc., of a disease or disorder, such as a carcinoma.

### Methods of treating angiogenic disorders

Also described are methods of treating disorders and diseases associated with excessive and/or abnormal angiogenesis.

Inappropriate and ectopic expression of angiogenesis can be deleterious to an organism. A number of pathological conditions are associated with the growth of extraneous blood vessels. These include, e.g., diabetic retinopathy, ischemic retinalvein occlusion, and retinopathy of prematurity (Aiello et al. New Engl. J. Med. 1994, 331, 1480; Peer et al. Lab. Invest 1995, 72, 638), age-related macular degeneration (AMD; see, Lopez et al. Invest. Opththalmol. Vis. Sci. 1996, 37, 855), neovascular glaucoma, psoriasis, retrolental fibroplasias, angiofibroma, inflammation, rheumatoid arthritis (RA), restenosis, in-stent restenosis, vascular graft restenosis, etc. In addition, the increased blood supply associated with cancerous and neoplastic tissue, encourages growth, leading to rapid tumor enlargement and metastasis. Moreover, the growth of new blood and lymph vessels in a tumor provides an escape route for renegade cells, encouraging metastasis and the consequence spread of the cancer. Thus, compounds of the present invention can be utilized to treat and/or prevent any of the aforementioned angiogenesis disorders, e.g., by inhibiting and/or reducing blood vessel formation; for example, by inhibiting, blocking, reducing, decreasing, endothelial cell proliferation or other types involved in angiogenesis, as well as causing cell death or apoptosis of such cell types.

Compound and compositions of the present invention can be tested routinely for angiogenic activity, e.g., by contacting a blood vessel-forming cell population with a compound of the present invention, and determining the effect of the compound on blood vessel formation. Any cell population capable of forming blood vessels can be utilized. Useful models, include, e.g., in vivo Matrigel-type assays; tumor neovascularization assays; CAM assays; BCE assays; cell migration assays; HUVEC growth inhibition assays; animal models (e.g., tumor growth in athymic mice, chronically ischemic lower limb in a rabbit model, cancer models, etc.); in vivo systems, such as a heart or limb present in a patient (e.g., angiogenic therapy to treat myocardial infarction); hosts in need of treatment, e.g., hosts suffering from angiogenesis related diseases, such as cancer, ischemic syndromes, arterial obstructive disease, to promote collateral circulation, to promote vessel growth into bioengineered tissues, etc.

Cells can include, e.g., endothelial, epithelial, muscle, embryonic and adult stem cells, ectodermal, mesenchymal, endodermal, neoplastic, blood, bovine CPAE (CCL-209), bovine FBHE (CRL-1395), human HUV-EC-C (CRL-1730), mouse SVEC4-10EHR1 (CRL-2161), mouse MS1 (CRL-2279), mouse MS1 VEGF (CRL-2460), stem cells, etc. The phrase "capable of forming blood vessels" does not indicate a particular cell-type, but simply that the cells in the population are able under appropriate conditions to form blood vessels. In some circumstances, the population may be heterogeneous, including more than one cell-type, only some which actually differentiate into blood vessels, but others which are necessary to initiate, maintain, etc., the process of vessel formation.

A useful model to determine the effect of compounds or compositions on angiogenesis is based on the observation that, when a reconstituted basement membrane matrix, such as Matrigel, supplemented with growth factor (e.g., FGF-1), is injected subcutaneously into a host animal, endothelial cells are recruited into the matrix, forming new blood vessels over a period of several days. See, e.g., Passaniti et al., Lab. Invest., 67:519-528, 1992. To stabilize the growth factor and/or slow its release from the matrix, the growth factor can be bound to heparin or another stabilizing agent. The matrix can also be periodically re-infused with growth factor to enhance and extend the angiogenic process. More specifically, a Matrigel plug implant including FGF-1 can be implanted subcutaneously into a host mouse. The initial bolus of FGF attracts endothelial cells into the implant, but does not result in new blood vessel formation. After about 10-15 days, the implant can be re-infused with FGF-1. The FGF-1 stimulates the endothelial cells already present in the implant, initiating the process of angiogenesis.

Other useful systems for studying angiogenesis, include, e.g., neovascularization of tumor explants (e.g., U.S. Pat. Nos. 5,192,744; 6,024,688), chicken chorioallantoic membrane (CAM) assay (e.g., Taylor and Folkman, Nature, 297:307-312, 1982; Eliceiri et al., J. Cell Biol., 140, 1255-1263, 1998), bovine capillary endothelial (BCE) cell assay (e.g., U.S. Pat. No. 6,024,688; Polverini, P. J. et al., Methods Enzymol., 198: 440-450, 1991), migration assays, HUVEC (human umbilical cord vascular endothelial cell) growth inhibition assay (e.g., U.S. Pat. No. 6,060,449).

A cell population can be contacted with the compound or composition in any manner and under any conditions suitable for it to exert an effect on the cells. The means by which compound is delivered to the cells may depend upon the type of test agent, e.g., its chemical nature, and the nature of the cell population.

Generally, a compound must have access to the cell population, so it must be delivered in a form (or pro-form) that the population can experience physiologically, i.e., to put in contact with the cells. For instance, if the intent is for the agent to enter the cell, if necessary, it can be associated with any means that facilitate or enhance cell penetrance, e.g., associated with antibodies or other reagents specific for cell-surface antigens, liposomes, lipids, chelating agents, targeting moieties, etc. Cells can also be treated, manipulated, etc., to enhance delivery, e.g., by electroporation, pressure variation, etc.

Based upon standard laboratory techniques known to evaluate compounds useful for the treatment of hyper-proliferative disorders and angiogenic disorders, by standard toxicity tests and by standard pharmacological assays for the determination of treatment of the conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and preferably from about 0.01 mg/kg to about 20 mg/kg body weight per day.

Clinically useful dosing schedules will range from one to three times a day dosing to once every four weeks dosing. In addition, "drug holidays" in which a patient is not dosed with a drug for a certain period of time, may be beneficial to the overall balance between pharmacological effect and tolerability. A unit dosage may contain from about 0.5 mg to about 1500 mg of active ingredient, and can be administered one or more times per day or less than once a day.

The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight.

The average daily topical dosage regimen will preferably be from 0.1 to 200 mg administered between one to four times daily. The transdermal concentration will preferably be that required to maintain a daily dose of from 0.01 to 200 mg/kg.

The average daily inhalation dosage regimen will preferably be from 0.01 to 100 mg/kg of total body weight.

The average daily oral dosage regimen will preferably be from 0.01 to 200 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like.

The desired mode of treatment and number of doses of a compound of the present invention or a pharmaceutically acceptable salt or ester or composition thereof can be ascertained by those skilled in the art using conventional treatment tests.

Accordingly, the present invention provides a method of treating hyper-proliferative disorders including administering to a mammal in need of treatment a therapeutically effective amount of one or more compounds according to the present invention.

The compounds according to the present invention are generically represented by the formula: 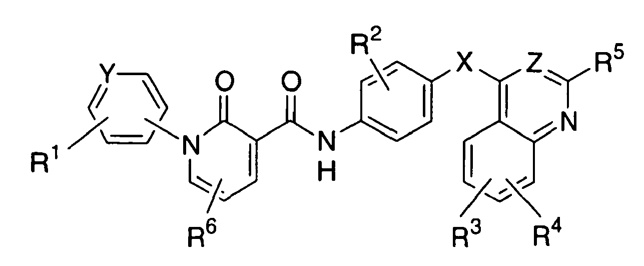 wherein the various groups are as previously defined herein above.

Pharmaceutically acceptable salts thereof, metabolites thereof, solvates thereof, hydrates thereof, prodrugs thereof, polymorphs thereof and diastereoisomeric forms thereof including isolated stereoisomers thereof and those within a mixture of stereoisomers are also included in the present invention.

Examples of the specific compounds of the present invention include compounds described in examples 1-7, as well as, salts, metabolites, solvates, hydrates and prodrugs thereof, including polymorphs and diastereoisomeric forms (both isolated stereoisomers and mixtures of stereoisomers) and combinations thereof.

The compounds of this invention can be administered as the sole pharmaceutical agent or in combination with one or more other pharmaceutical agents where the combination causes no unacceptable adverse effects. For example, the compounds of this invention can be combined with known anti-hyper-proliferative or other indication agents, and the like, as well as with admixtures and combinations thereof.

Optional anti-hyper-proliferative agents which can be added to the composition include but are not limited to compounds listed on the cancer chemotherapy drug regimens in the 11th Edition of the Merck Index, (1996), such as asparaginase, bleomycin, carboplatin, carmustine, chlorambucil, cisplatin, colaspase, cyclophosphamide, cytarabine, dacarbazine, dactinomycin, daunorubicin, doxorubicin (adriamycine), epirubicin, etoposide, 5-fluorouracil, hexamethylmelamine, hydroxyurea, ifosfamide, irinotecan, leucovorin, lomustine, mechlorethamine, 6-mercaptopurine, mesna, methotrexate, mitomycin C, mitoxantrone, prednisolone, prednisone, procarbazine, raloxifen, streptozocin, tamoxifen, thioguanine, topotecan, vinblastine, vincristine, and vindesine.

Other anti-hyper-proliferative agents suitable for use with the composition of the invention include but are not limited to those compounds acknowledged to be used in the treatment of neoplastic diseases in Goodman and Gilman's The Pharmacological Basis of Therapeutics (Ninth Edition), editor Molinoff et al., published by McGraw-Hill, pages 1225-1287, (1996), such as aminoglutethimide, L-asparaginase, azathioprine, 5-azacytidine cladribine, busulfan, diethylstilbestrol, 2',2'-difluorodeoxycytidine, docetaxel, erythrohydroxynonyl adenine, ethinyl estradiol, 5-fluorodeoxyuridine, 5-fluorodeoxyuridine monophosphate, fludarabine phosphate, fluoxymesterone, flutamide, hydroxyprogesterone caproate, idarubicin, interferon, medroxyprogesterone acetate, megestrol acetate, melphalan, mitotane, paclitaxel, pentostatin, N-phosphonoacetyl-L-aspartate (PALA), plicamycin, semustine, teniposide, testosterone propionate, thiotepa, trimethylmelamine, uridine, and vinorelbine.

Other anti-hyper-proliferative agents suitable for use with the composition of the invention include but are not limited to other anti-cancer agents such as epothilone and its derivatives, irinotecan, raloxifen and topotecan.

The compounds of this invention can be administered as the sole pharmaceutical agent or in combination with one or more other pharmaceutical agents where the combination causes no unacceptable adverse effects. For example, the compounds of this invention can be combined with known anti-hyper-proliferative or other indication agents, and the like, as well as with admixtures and combinations thereof.

Optional anti-hyper-proliferative agents which can be added to the composition include but are not limited to compounds listed on the cancer chemotherapy drug regimens in the 11th Edition of the Merck Index, (1996), which is hereby incorporated by reference, such as asparaginase, bleomycin, carboplatin, carmustine, chlorambucil, cisplatin, colaspase, cyclophosphamide, cytarabine, dacarbazine, dactinomycin, daunorubicin, doxorubicin (adriamycine), epirubicin, etoposide, 5-fluorouracil, hexamethylmelamine, hydroxyurea, ifosfamide, irinotecan, leucovorin, lomustine, mechlorethamine, 6-mercaptopurine, mesna, methotrexate, mitomycin C, mitoxantrone, prednisolone, prednisone, procarbazine, raloxifen, streptozocin, tamoxifen, thioguanine, topotecan, vinblastine, vincristine, and vindesine.

Other anti-hyper-proliferative agents suitable for use with the composition of the invention include but are not limited to those compounds acknowledged to be used in the treatment of neoplastic diseases in Goodman and Gilman's The Pharmacological Basis of Therapeutics (Ninth Edition), editor Molinoff et al., published by McGraw-Hill, pages 1225-1287, (1996) , such as aminoglutethimide, L-asparaginase, azathioprine, 5-azacytidine cladribine, busulfan, diethylstilbestrol, 2',2'-difluorodeoxycytidine, docetaxel, erythrohydroxynonyl adenine, ethinyl estradiol, 5-fluorodeoxyuridine, 5-fluorodeoxyuridine monophosphate, fludarabine phosphate, fluoxymesterone, flutamide, hydroxyprogesterone caproate, idarubicin, interferon, medroxyprogesterone acetate, megestrol acetate, melphalan, mitotane, paclitaxel, pentostatin, N-phosphonoacetyl-L-aspartate (PALA), plicamycin, semustine, teniposide, testosterone propionate, thiotepa, trimethylmelamine, uridine, and vinorelbine.

Other anti-hyper-proliferative agents suitable for use with the composition of the invention include but are not limited to other anti-cancer agents such as epothilone and its derivatives, irinotecan, raloxifen and topotecan.

Generally, the use of cytotoxic and/or cytostatic agents in combination with a compound or composition of the present invention will serve to:
(1) yield better efficacy in reducing the growth of a tumor or even eliminate the tumor as compared to administration of either agent alone;
(2) provide for the administration of lesser amounts of the administered chemotherapeutic agents;
(3) provide for a chemotherapeutic treatment that is well tolerated in the patient with fewer deleterious pharmacological complications than observed with single agent chemotherapies and certain other combined therapies;
(4) provide for treating a broader spectrum of different cancer types in mammals, especially humans;
(5) provide for a higher response rate among treated patients;
(6) provide for a longer survival time among treated patients compared to standard chemotherapy treatments; and
(7) provide a longer time for tumor progression, and/or yield efficacy and tolerability results at least as good as those of the agents used alone, compared to known instances where other cancer agent combinations produce antagonistic effects.

The pharmaceutical composition can further include an additional anti-hyper-proliferative agent and/or an additional pharmaceutical agent.

The additional anti-hyper-proliferative agent can be a compound, such as, epothiline or a derivative thereof, irinotecan, raloxifen or topotecan. The additional pharmaceutical agent can be aldesleukin, alendronic acid, alfaferone, alitretinoin, allopurinol, aloprim, aloxi, altretamine, aminoglutethimide, amifostine, amrubicin, amsacrine, anastrozole, anzmet, aranesp, arglabin, arsenic trioxide, aromasin, 5-azacytidine, azathioprine, BCG or tice BCG, bestatin, betamethasone acetate, betamethasone sodium phosphate, bexarotene, bleomycin sulfate, broxuridine, bortezomib, busulfan, calcitonin, campath, capecitabine, carboplatin, casodex, cefesone, celmoleukin, cerubidine, chlorambucil, cisplatin, cladribine, cladribine, clodronic acid, cyclophosphamide, cytarabine, dacarbazine, dactinomycin, DaunoXome, decadron, decadron phosphate, delestrogen, denileukin diftitox, depomedrol, deslorelin, dexrazoxane, diethylstilbestrol, diflucan, docetaxel, doxifluridine, doxorubicin, dronabinol, DW-166HC, eligard, elitek, ellence, emend, epirubicin, epoetin alfa, epogen, eptaplatin, ergamisol, estrace, estradiol, estramustine phosphate sodium, ethinyl estradiol, ethyol, etidronic acid, etopophos, etoposide, fadrozole, farston, filgrastim, finasteride, fligrastim, floxuridine, fluconazole, fludarabine, 5-fluorodeoxyuridine monophosphate, 5-fluorouracil (5-FU), fluoxymesterone, flutamide, formestane, fosteabine, fotemustine, fulvestrant, gammagard, gemcitabine, gemtuzumab, gleevec, gliadel, goseretin, granisetron HCl, histrelin, hycamtin, hydrocortone, eyrthro-hydroxynonyladenine, hydroxyurea, ibritumomab tiuxetan, idarubicin, ifosfamide, interferon alpha, interferon-alpha 2, interferon alfa-2A, interferon alfa-2B, interferon alfa-n1, interferon alfa-n3, interferon beta, interferon gamma-1a, interleukin-2, intron A, iressa, irinotecan, kytril, lentinan sulphate, letrozole, leucovorin, leuprolide, leuprolide acetate, levamisole, levofolinic acid calcium salt, levothroid, levoxyl, lomustine, lonidamine, marinol, mechlorethamine, mecobalamin, medroxyprogesterone acetate, megestrol acetate, melphalan, menest, 6-mercaptopurine, Mesna, methotrexate, metvix, miltefosine, minocycline, mitomycin C, mitotane, mitoxantrone, Modrenal, Myocet, nedaplatin, neulasta, neumega, neupogen, nilutamide, nolvadex, NSC-631570, OCT-43, octreotide, ondansetron HCl, orapred, oxaliplatin, paclitaxel, pediapred, pegaspargase, Pegasys, pentostatin, picibanil, pilocarpine HCl, pirarubicin, plicamycin, porfimer sodium, prednimustine, prednisolone, prednisone, premarin, procarbazine, procrit, raltitrexed, rebif, rhenium-186 etidronate, rituximab, roferon-A, romurtide, salagen, sandostatin, sargramostim, semustine, sizofiran, sobuzoxane, solu-medrol, sparfosic acid, stem-cell therapy, streptozocin, strontium-89 chloride, sutent, synthroid, tamoxifen, tamsulosin, tasonermin, tastolactone, taxotere, teceleukin, temozolomide, teniposide, testosterone propionate, testred, thioguanine, thiotepa, thyrotropin, tiludronic acid, topotecan, toremifene, tositumomab, trastuzumab, treosulfan, tretinoin, trexall, trimethylmelamine, trimetrexate, triptorelin acetate, triptorelin pamoate, UFT, uridine, valrubicin, vesnarinone, vinblastine, vincristine, vindesine, vinorelbine, virulizin, zinecard, zinostatin stimalamer, zofran, ABI-007, acolbifene, actimmune, affinitak, aminopterin, arzoxifene, asoprisnil, atamestane, atrasentan, sorafenib, avastin, CCI-779, CDC-501, celebrex, cetuximab, crisnatol, cyproterone acetate, decitabine, DN-101, doxorubicin-MTC, dSLIM, dutasteride, edotecarin, eflornithine, exatecan, fenretinide, histamine dihydrochloride, histrelin hydrogel implant, holmium-166 DOTMP, ibandronic acid, interferon gamma, intron-PEG, ixabepilone, keyhole limpet hemocyanin, L-651582, lanreotide, lasofoxifene, libra, lonafarnib, miproxifene, minodronate, MS-209, liposomal MTP-PE, MX-6, nafarelin, nemorubicin, neovastat, nolatrexed, oblimersen, onco-TCS, osidem, paclitaxel polyglutamate, pamidronate disodium, PN-401, QS-21, quazepam, R-1549, raloxifene, ranpirnase, 13-cis -retinoic acid, satraplatin, seocalcitol, T-138067, tarceva, taxoprexin, thymosin alpha 1, tiazofurine, tipifamib, tirapazamine, TLK-286, toremifene, TransMID-107R, valspodar, vapreotide, vatalanib, verteporfin, vinflunine, Z-100, zoledronic acid, or combinations thereof.

### General Preparative Methods

In general, the compounds of this invention may be prepared by standard techniques known in the art, by known processes analogous thereto, and/or by the processes described herein, using starting materials that are either commercially available or that can be produced according to routine, conventional chemical methods. One skilled in the art will recognize that modifications may be made in the present invention without deviating from the spirit or scope of the invention.

The present invention is further illustrated by the following experimental information and examples, which are not to be construed as limiting the invention in spirit or scope to the specific procedures or compositions described in them. Intermediates employed in the synthesis of compounds of this invention may possess sensitive or reactive functional groups that may need to be protected during any of the methods described below. Removal of such protective groups is completed at appropriate stages by methods well known to those skilled in the art (*see, e.g.*, T. W. Greene and P.G.M. Wuts, Protective Groups in Organic Synthesis; Wiley: New York, (1999)).

The specific preparative method to be employed in the synthesis of the compounds of this invention is dependent upon the particular compound desired. Factors such as the identity of the X group and the specific substituents present in the molecule, all play a role in the selection of the path used to prepare the specific compounds of this invention. Those factors are readily recognized by one of ordinary skill in the art.

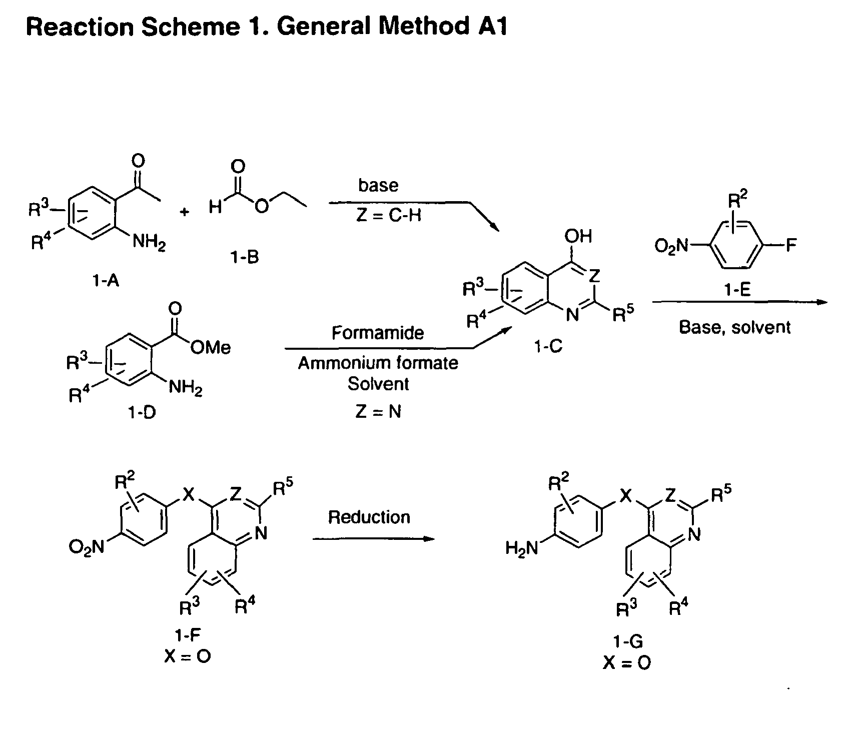

The compounds 1-G are synthesized according to the reaction scheme outlined in General Method A1. Treatment of anilino-ketones (1-A) with ethyl formate in presence of a base, preferably sodium ethoxide, in a solvent such as ethanol provides compounds 1-C, where X = CH.

Similarly, treatment of anilino-benzoates 1-D with ammonium formate in formamide, either neat or with a solvent, produces the quinazoline derivatives 1-C, where X = N. Addition of the compounds 1-C to 4-fluoro nitrobenzene compounds 1-E in the presence of a base, such as cesium or potassium carbonate, in a solvent such as DMF or acetonitrile, provides intermediates of structure 1-F. Hydrogenation of the compounds 1-F in the presence of a suitable catalyst, such as palladium on carbon, provides the compounds 1-G.

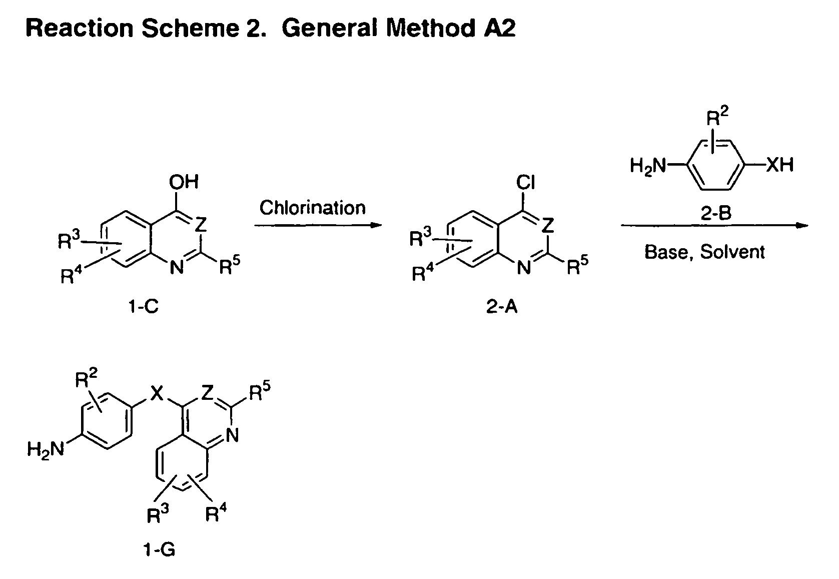

Alternatively, the compounds 1-G are synthesized according to the method shown in General Method A2.

The compounds 1-C (see Scheme 1) upon treatment with chlorinating agents, such as phosphorus oxychloride or thionyl chloride, furnish the compounds 2-A.

Subsequent coupling of compounds 2-A with compounds 2-B in the presence of a base such as sodium hydride and in a solvent such as tetrahydrofuran provides the compounds 1-G.

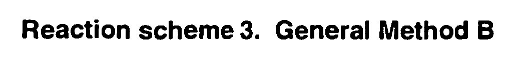

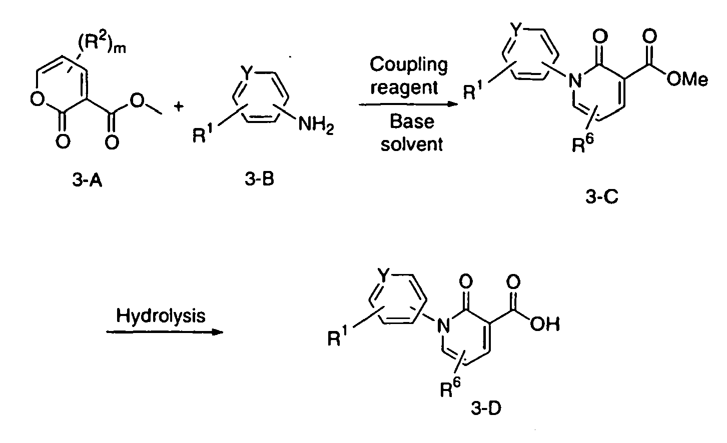

The compounds 3-D are synthesized according to the pathway outlined in General Method B above.

Pyranones 3-A can be reacted with amines 3-B in the presence of a coupling reagent such as EDCI in a suitable solvent such as THF to furnish the compounds 3-C.

This reaction can be further catalyzed by additional reagents such as DMAP. Treatment of the compounds 3-C with a hydroxide source, such as NaOH or KOH, in a solvent or mixture of suitable solvents, such as methanol and water, provides the compounds 3-D.

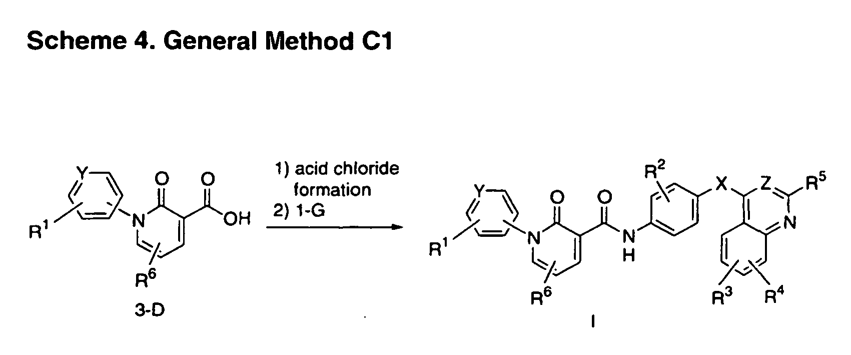

Compounds of Formula I are synthesized according to the General Method C1 shown above. The compounds 3-D are first converted to the corresponding acid chloride using a chlorinating agent, such as phosphorus oxychloride or thionyl chloride. Subsequent reaction of the acid chloride thus produced with the compounds 1-G in the presence of an organic base, such as Et₃N and a catalyst such as DMAP, in a suitable solvent, such as THF provides the compounds of Formula I.

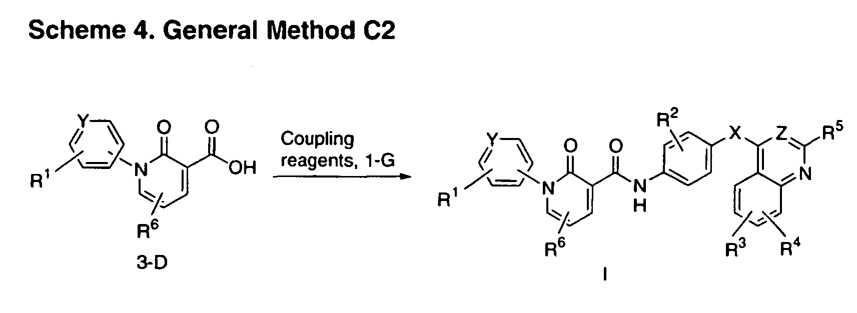

Alternatively, compounds of Formula I can be produced according to the General Method C2. The compounds 3-D are coupled with 1-G in a suitable solvent, such as THF, in the presence of an activating reagent, such as EDCI, in the presence of base, preferably Et₃N, and optionally in the presence of a catalyst, such as HOBT or DMAP, to provide compounds of Formula I.

### Abbreviations and Acronyms

A comprehensive list of the abbreviations utilized by organic chemists of ordinary skill in the art appears in the first issue of each volume of the *Journal of Organic Chemistry*; this list is typically presented in a table entitled Standard List of Abbreviations. The abbreviations contained in said list, and all abbreviations utilized by organic chemists of ordinary skill in the art are hereby incorporated by reference.

For purposes of this invention, the chemical elements are identified in accordance with the Periodic Table of the Elements, CAS version, Handbook of Chemistry and Physics, 67th Ed., 1986-87.

More specifically, when the following abbreviations are used throughout this disclosure, they have the following meaning:

### Abbreviations

- ¹H NMR: proton nuclear magnetic resonance
- Ac: acetyl
- AcOH: acetic acid
- amu: atomic mass unit
- aq: aqueous
- atm: atmosphere
- ATP: Adenosine triphosphate
- BRIJ: Polyoxyethylene(23) dodecyl ether
- bs: broad singlet
- BSA: bovine serum albumin
- Bu: butyl
- Celite®: brand of diatomaceous earth filtering agent, registered trademark of Celite Corporation
- conc: concentration
- d: doublet
- DCE: 1,2-Dichloroethane
- DCM: Dichloromethane
- dd: doublet of doublet
- ddd: doublet of doublet of doublet
- DMAP: 4-(N,N-dimethylamino)pyridine
- DME: dimethyoxyethane
- DMF: N,N-Dimethyl formamide
- DMSO: Dimethyl sulphoxide
- EDCI: 1-[3-(Dimethylamino) propyl]-3-ethylcarbodiimide hydrochloride
- EDTA: ethylene diamine tetraacetate
- EI-MS: Electron impact mass spectroscopy
- ELSD: Evaporative Light Scattering Detector
- ES: Electrospray
- ES-MS: Electrospray mass spectroscopy
- Et: ethyl
- Et₃N: Triethylamine
- EtOAc: Ethyl acetate
- EtOH: Ethanol
- Ex: example
- g: gram
- h: hour(s)
- HEPES: N-(2-hydroxyethyl)-piperazine-N'-(2-ethane sulfonic acid)
- Hex: hexanes
- HOBT: 1-Hydroxybenzotriazole
- HPLC: High pressure liquid chromatography
- KO*t*Bu: potassium tert-butoxide
- L: liter
- LCMS: Liquid chromatography - coupled mass spectroscopy
- m: multiplet
- M: molar
- m/z: mass over charge
- Me: methyl
- MeCN: acetonitrile
- MeOH: Methanol
- mg: milligram
- MHz: megahertz
- min: minute(s)
- mL: milliliter
- mm: millimeter
- mmol: millimole
- mol: mole
- MPLC: Medium pressure liquid chromatography
- N: Normal
- NaOEt: Sodium ethoxide
- NaOMe: Sodium methoxide
- NMR: Nuclear resonance spectroscopy
- PBS: phosphate-buffered saline
- Ph: phenyl
- ppm: parts per million
- Pr: propyl
- psi: pounds per square inch
- q: quartet
- rt: room temperature
- RT: retention time (HPLC)
- s: singlet
- t: triplet
- TFA: trifluoroacetic acid
- THF: Tetrahydrofuran
- TLC: Thin layer chromatography
- TMB: Tetramethylbenzidine
- Tris: tris(hydroxymethyl)-aminomethane

The percentage yields reported in the following examples are based on the starting component that was used in the lowest molar amount. Air and moisture sensitive liquids and solutions were transferred via syringe or cannula, and introduced into reaction vessels through rubber septa. Commercial grade reagents and solvents were used without further purification. The term "concentrated under reduced pressure" refers to use of a Buchi rotary evaporator at approximately 15 mm of Hg. All temperatures are reported uncorrected in degrees Celsius (°C). Thin layer chromatography (TLC) was performed on pre-coated glass-backed silica gel 60 A F-254 250 µm plates

Electron impact mass spectra (EI-MS) were obtained with a Hewlett Packard 5989A mass spectrometer equipped with a Hewlett Packard 5890 Gas Chromatograph with a J & W DB-5 column (0.25 µM coating; 30 m x 0.25 mm). The ion source was maintained at 250°C and spectra were scanned from 50-800 amu at 2 sec per scan.

High pressure liquid chromatography-electrospray mass spectra (LC-MS) were obtained using either a:
a) Hewlett-Packard 1100 HPLC equipped with a quaternary pump, a variable wavelength detector set at 254 nm, a YMC pro C-18 column (2 x 23 mm, 120A), and a Finnigan LCQ ion trap mass spectrometer with electrospray ionization. Spectra were scanned from 120-1200 amu using a variable ion time according to the number of ions in the source. The eluents were A: 2% acetonitrile in water with 0.02% TFA, and B: 2% water in acetonitrile with 0.018% TFA. Gradient elution from 10% to 95% B over 3.5 minutes at a flow rate of 1.0 mL/min was used with an initial hold of 0.5 minutes and a final hold at 95% B of 0.5 minutes. Total run time was 6.5 minutes.
b) Gilson HPLC system equipped with two Gilson 306 pumps, a Gilson 215 Autosampler, a Gilson diode array detector, a YMC Pro C-18 column (2 x 23mm, 120 A), and a Micromass LCZ single quadrupole mass spectrometer with z-spray electrospray ionization. Spectra were scanned from 120-800 amu over 1.5 seconds. ELSD (Evaporative Light Scattering Detector) data was also acquired as an analog channel. The eluents were A: 2% acetonitrile in water with 0.02% TFA, and B: 2% water in acetonitrile with 0.018% TFA. Gradient elution from 10% to 90% B over 3.5 minutes at a flow rate of 1.5 mL/min was used with an initial hold of 0.5 minutes and a final hold at 90% B of 0.5 minutes. Total run time was 4.8 minutes. An extra switching valve was used for column switching and regeneration.
c) Agilent 1100 HPLC system. The Agilent 1100 HPLC system was equipped with an Agilent 1100 autosampler, quaternary pump, and a diode array.
The HPLC column used was a Waters Sunfire (2.1 x 30 mm, 3.5 uM). The HPLC eluent was directly coupled with a 1:4 split to a Finnigan LTQ ion trap mass spectrometer with electrospray ionization. Spectra were scanned from 50-1000 amu using a variable ion time according to the number of ions in the source in either positive or negative ion mode. The eluents were A: water with 0.1 Formic acid and B: acetonitrile with 0.1 % Formic acid. Gradient elution from 10% B to 90% B over 3.0 minutes at a flowrate of 1.0 mL/min was used with an initial hold of 2.0 minutes and a final hold at 95% B of 1.0 minutes. Total run time was 8.0 minutes.

Routine one-dimensional NMR spectroscopy was performed on 300/400 MHz Varian Mercury-plus spectrometers. The samples were dissolved in deuterated solvents obtained from Cambridge Isotope Labs, and transferred to 5mm ID Wilmad NMR tubes. The spectra were acquired at 293 K. The chemical shifts were recorded on the ppm scale and were referenced to the appropriate solvent signals, such as 2.05 ppm for acetone-*d*₆, 2.49 ppm for DMSO-*d*₆, 1.93 ppm for CD₃CN, 3.30 ppm for CD₃OD, 5.32 ppm for CD₂Cl₂ and 7.26 ppm for CDCl₃ for ¹H spectra.

### Preparative HPLC

Preparative HPLC was carried out in reversed phase mode, eluting with aqueous acetonitrile containing 0.5% TFA, typically using a Gilson HPLC system equipped with two Gilson 322 pumps, a Gilson 215 Autosampler, a Gilson diode array detector, and a YMC Pro C-18 column (20 x 150 mm, 120 A). Gradient elution was used with Buffer A as water with 0.1 % TFA and Buffer B as acetonitrile with 0.1 % TFA.

The sample was dissolved in MeOH or MeOH/DMSO with concentration about 50 mg/mL. Injection volume was about 2-3 mL/injection. Sample was typically eluted as follows: 10-90% B over 15 minutes with flow rate of 25 mL/min, hold 2 minutes, back to 10% B. The desired fraction(s) were collected by UV monitoring at 254 or 220 nm and evaporated by using a GeneVac centrifugal vacuum instrument.

### Preparative MPLC or silica gel chromatography:

Preparative medium pressure liquid chromatography (MPLC) was carried out by standard silica gel "flash chromatography" techniques (e.g., Still, W. C. et al. J. Org. Chem. 1978, 43, 2923-5), or by using silica gel cartridges and devices such as the Biotage Flash systems. A variety of eluting solvents were used, as described in the experimental protocols. By using these above described methods, the compounds of the invention may be prepared.

The compounds described in the examples are intended to be representative of the invention, and it will be understood that the scope of the invention is not limited by the scope of the examples.

The following specific examples are presented to further illustrate the invention described herein, but they should not be construed as being limiting the scope of the invention in any way.

### Intermediate A

### Preparation of of 4-[(6,7-dimethoxyquinolin-4-yl)oxy]aniline

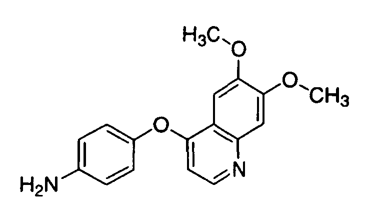

### Step 1: Preparation of 6,7-dimethoxyquinolin-4-ol:

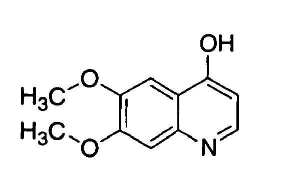

To a solution of 2-amino-4,5-dimethoxylacetophenone (4.5 g, 23 mmol) in DME (100 mL) was added NaOMe (5.0 g, 92 mmol). The reaction was stirred for 30 min after which ethyl formate (8.5 g, 115 mmol) was added and the reaction mixture was stirred for 16 h at rt. Then NaOEt (21% dispersion in oil, 22 g, 69.1 mmol) was added and the reaction mixture was stirred for an additional 24 h at rt. Water (10mL) was then added, and the mixture was stirred for 1 h, then concentrated under reduced pressure. The pH of the resulting aqueous mixture was adjusted to pH 7 using HCl (2N aqueous solution). The resulting precipitated was collected by filtration, and then successively washed with water, ethyl acetate and ethyl ether. The solid was dried under mechanical vacuum give 2.6 g (yield 55 %) of the title compound.
¹H NMR (400 MHz, DMSO-*d₆*) δ 10.97 (bs, 1 H), 7.79 (d, 1 H), 7.42 (s, 1 H), 6.96 (s, 1 H), 5.96 (d, 1 H), 3.84 (s, 3 H), 3.81 (s, 3 H); ES-MS *m*/*z* 206.2 [M+H]⁺, LCMS RT (min) 1.21.

### Step 2. Preparation of 6,7-dimethoxy-4-(4-nitrophenoxy)quinoline

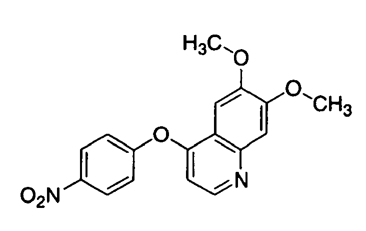

A solution of 1-fluoro-4-nitrobenzene (0.83 g, 5.8 mmol) in a mixture of DMF (7.5 mL) and CH₃CN (7.5 mL) was added Cs₂CO₃ (3.4 g, 10.6 mmol) was stirred for 30 min, then 6,7-dimethoxyquinolin-4-ol (1.09 g, 5.3mmol) was added and the reaction mixture was stirred at 60°C for 16 h. The reaction mixture was cooled to rt and then concentrated under reduced pressure. Water (20 mL) was added to the residue and the mixture was extracted with ethyl acetate. The combined organic layers were washed successively with water and brine, dried (Na₂SO₄), filtered and concentrated under reduced pressure. The residue was purified by silica gel flash chromatography (eluent: 50 to 100% ethyl acetate in hexane) to give 504 mg of desired compound. (yield 31 %) of the desired product.
¹H NMR (400 MHz, DMSO-*d₆*) δ 8.60 (d, 1 H), 8.33 (d, 2 H), 7.44 (s, 1 H), 7.42 (d, 2 H), 7.35 (s, 1 H), 6.86 (d, 1 H), 3.94 (s, 3 H), 3.87 (s, 3 H); ES-MS *m*/*z* 327.2 [M+H]⁺, LCMS RT (min) 2.28.

### Step 3. Preparation of 4-[(6,7-dimethoxyquinolin-4-yl)oxy]aniline

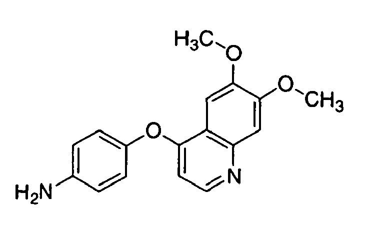

To a round bottom flask equipped with stirring bar were added 6,7-dimethoxy-4-(4-nitrophenoxy)quinoline (534 mg, 1.64 mmol), palladium over carbon (10%, 35 mg), EtOH (40mL) and DMF (20mL). The reaction mixture was placed under an atmosphere of H₂ (1 atm) and stirred for 16 h. The H₂ was thoroughly evacuated from the reaction vessel, and then the reaction mixture was filtered over Celite®. The filtrate was concentrated under reduced pressure, and the residue was carried into the next step without further purification. An aliquot was isolated and characterized by ¹H NMR. ¹H NMR (300 MHz, DMSO-*d₆*) δ 8.41 (d, 1 H), 7.48 (s, 1H), 7.34 (s, 1 H), 6.90 (d, 2 H), 6.64 (d, 2 H), 6.34 (d, 1 H), 5.15 (bs, 2 H), 3.92 (s, 3 H), 3.91 (s, 3 H); ES-MS *m*/*z* 297 [M+H]⁺, LCMS RT (min) 1.00.

### Intermediate B

### Preparation of 4-[(6,7-dimethoxyquinolin-4-yl)oxy]-2-fluoroaniline

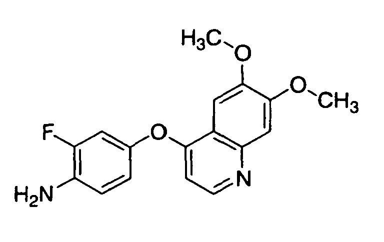

### Step 1. Preparation of 4-chloro-6,7-dimethoxyquinoline

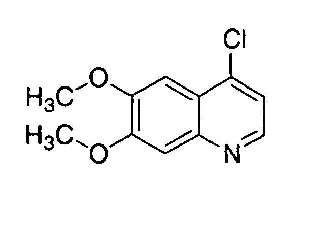

To a mixture 6,7-dimethoxyquinolin-4-ol (**Intermediate A, Step 2,** 245 mg, 1.2 mmol) in phosphorus oxychloride (10 mL) was added DMF (0.1mL). The reaction was heated at reflux for 4 h, then cooled to rt and concentrated under reduced pressure. The residue was dissolved with ethyl acetate, and the organic solution was washed successively with NaHCO₃ (saturated aqueous solution) and water, dried (Na₂SO₄), filtered and concentrated under reduced pressure to give 116 mg (43.4%) of title compound, which was taken onto next step without further purification.

### Step 2. Preparation of 4-[(6,7-dimethoxyquinolin-4-yl)oxy]-2-fluoroaniline

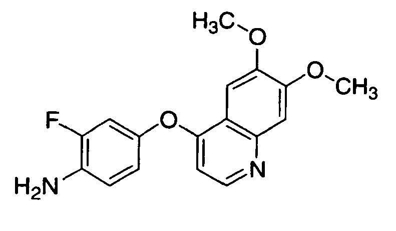

To a solution of 4-amino-3-fluorophenol (65.9mg, 0.52 mmol) in DMSO (10mL) was added KOt-Bu (84 mg, 0.75 mmol) and the reaction mixture was stirred at rt for 30 min. Then 4-chloro-6,7-dimethoxyquinoline (116 mg, 0.519 mmol) was added, and the reaction mixture was stirred at 90°C for an additional 16 h. The reaction mixture was cooled to rt and diluted with ethyl acetate. The organic mixture was then washed successively with water and K₂CO₃ (10% aqueous solution), dried (Na₂SO₄), filtered and concentrated under reduced pressure. The residue was purified by silica gel flash chromatography (eluent: 50 to 100% ethyl acetate in hexanes) to give 50 mg (31 %) of the title compound. ¹H NMR (400 MHz, CD₃CN) δ 8.45 (d, 1 H), 7.57 (s, 1 H), 7.39 (s, 1 H), 6.98-6.86 (m, 3 H), 6.49 (d, 1 H), 4.27 (bs, 2 H), 3.99 (s, 3 H), 3.97 (s, 3 H); ES-MS *m*/*z* 315.3 [M+H]⁺, LCMS RT (min) 2.08.

### Intermediate C

### Preparation of 4-[(6,7-dimethoxyquinazolin-4-yl)oxy]aniline

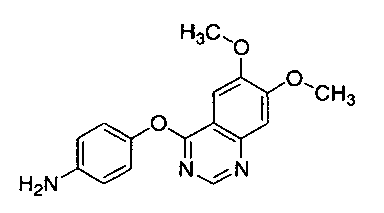

### Step 1. Preparation of 6,7-dimethoxyquinazolin-4-ol

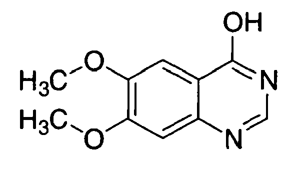

To a solution of methyl 2-amino-4,5-dimethoxybenzoate (5.0 g, 23.7 mmol) in formamide (50 mL) was added ammonium formate (2.2 g, 35.3 mmol). The reaction mixture was stirred at 150°C for 4 h, cooled to rt, and then poured into water. The resulting precipitate was collected by filtration, washed with water and dried under mechanical vacuum 4.5 g of desired product. ¹H NMR (400 MHz, DMSO-*d₆*) δ 12.07 (s, 1 H), 7.97 (s, 1 H), 7.42 (s, 1 H), 7.12 (s, 1 H), 3.88 (s, 3 H), 3.85 (s, 3 H); ES-MS *m*/*z* 206.8 [M+H]⁺, LCMS RT (min) 1.37.

### Step 2. Preparation of 4-chloro-6,7-dimethoxyquinazoline

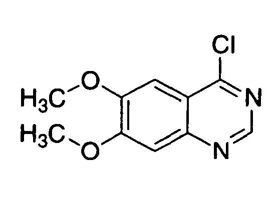

To a mixture of 6,7-dimethoxyquinazolin-4-ol (975 mg, 4.7 mmol) in thionyl chloride (5mL) was added DMF (0.1 mL). The reaction was heated at reflux for 4 h, then cooled to rt and concentrated under reduced pressure. The residue was dried under mechanical vacuum to give 1.2 g of the title compound. ¹H NMR (400 MHz, CD₃CN) δ 8.83 (s, 1 H), 7.47 (s, 1 H), 7.41 (s, 1 H), 4.06 (s, 3 H), 4.04 (s, 3 H); ES-MS *m*/*z* 225.1 [M+H]⁺, LCMS RT (min) 2.45.

### Step 3. Preparation of 4-[(6,7-dimethoxyquinazolin-4-yl)oxy]aniline

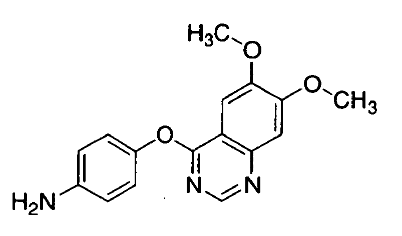

To a solution of 4-aminophenol (194 mg, 1.8 mmol) in DMF (10 mL) was added KOt-Bu (240 mg, 2.1 mmol) and the reaction mixture was stirred for 30 min. Then, 4-chloro-6,7-dimethoxyquinoline (400 mg, 1.8 mmol) was added and the reaction mixture was stirred at 80°C for an additional 16 h. The reaction mixture was then diluted with ethyl acetate, and the organic mixture was washed successively with water and K₂CO₃ (10% aqueous solution), dried (Na₂SO₄), filtered and concentrated under reduced pressure. The residue was triturated with ether to give 280 mg (yield 53%) of the title compound. ¹H NMR (400 MHz, CD₃CN) δ 8.50 (s, 1 H), 7.60 (s, 1 H), 7.32 (s, 1 H), 6.98 (d, 2 H), 6.74 (d, 2 H), 4.18 (bs, 2 H), 4.04 (s, 3 H), 4.01 (s, 3 H); ES-MS *m*/*z* 298.2 [M+H]⁺, LCMS RT (min) 1.76.

### Intermediate D

### Preparation of 4-[(6,7-dimethoxyquinazolin-4-yl)thio]-2-fluoroaniline

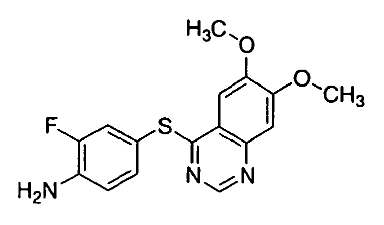

To a solution of 4-amino-3-fluorobenzenethiol (280 mg, 2.0 mmol) in DMF (10 mL) was added K₂CO₃ (738 mg, 5.3 mmol), and the reaction was stirred for 60 min at 50°C. Then, 4-chloro-6,7-dimethoxyquinoline (**Intermediate C, Step 2,** 400 mg, 1.8 mmol) was added and the reaction mixture was stirred at 50°C for 16 h. The reaction mixture cooled to rt and diluted with ethyl acetate. The organic mixture was washed successively with water, K₂CO₃ (10% aqueous solution) and NH₄Cl (10% aqueous solution), dried (Na₂SO₄), filtered and concentrated under reduced pressure. The residue was purified by silica gel flash chromatography (eluent: 50 to 100% ethyl acetate in hexanes) to give 320 mg (54%) of the title compound. ¹H NMR (400 MHz, CD₃CN) δ 8.62 (s, 1 H), 7.34 (s, 1 H), 7.30 (s, 1 H), 7.26 (dd, 1 H), 7.17 (dd, 1 H), 6.92 (dd, 1 H), 4.59 (bs, 2 H), 4.08 (s, 3 H), 4.01 (s, 3 H); ES-MS *m*/*z* 332.2 [M+H]⁺, LCMS RT (min) 2.72.

### Intermediate E

### Preparation of 1-(4-fluorophenyl)-2-oxo-1,2-dihydropyridine-3-carboxylic acid

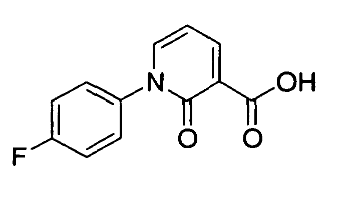

### Step 1. Preparation of methyl1-(4-fluorophenyl)-2-oxo-1,2-dihydropyridine-3-carboxylate

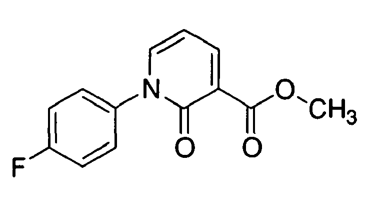

To a solution of methyl 2-oxo-2H-pyran-3-carboxylate (5g, 32.4 mmol) in THF (100mL) was added 4-fluoroaniline (3.6 g, 32.4 mmol), and the reaction mixture was stirred at rt for 2.5 h. Then 1-(3-dimethylaminopropyl)-3-ethylcarbodiimide hydrochloride (8.1 g, 42.2 mmol) and DMAP (0.4 g, 3.2 mmol) were added, and the mixture was stirred at rt for 16 h. The reaction mixture was then between HCl (1 N aqueous solution) and EtOAc. The organic phase was separated, and then washed successively with HCl (0.5 N aqueous solution), water and brine, dried (Na₂SO₄), filtered and concentrated under reduced pressure. The residue was purified by silica gel flash chromatography (eluent 20 to 50% ethyl acetate in hexanes) to give 4.4 g of the title compound. ¹H NMR (400 MHz, CD₃CN) δ 8.13 (dd, 1 H), 7.67 (dd, 1 H), 7.45-7.42 (m, 2 H), 7.30-7.25 (m, 2 H), 6.36 (t, 1 H), 3.81 (s, 3 H); ES-MS *m*/*z* 247.9 [M+H]⁺, LCMS RT (min) 2.27.

### Step 2. Preparation of 1-(4-fluorophenyl)-2-oxo-1,2-dihydropyridine-3-carboxylic acid

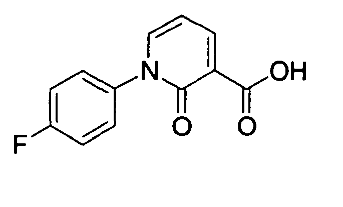

To a solution of methyl 1-(4-fluorophenyl)-2-oxo-1,2-dihydropyridine-3-carboxylate (4.4 g, 17.8 mmol) in methanol (100 mL) was added NaOH (1 N aqueous solution, 26.7 mL, 26.7 mmol). The reaction mixture was stirred at rt for 16 h, and then concentrated under reduced pressure to remove the methanol. The resulting aqueous mixture was acidified to pH 4 using HCl (1 N aqueous solution), and the resulting precipitate was collected by filtration. The solid was then washed successively with water and diethyl ether, and then dried under mechanical to give 2.9 g of the title compound. ¹H NMR (400 MHz, DMSO-*d₆*) δ 8.47 (dd, 1 H), 8.20 (dd, 1 H), 7.62-7.59 (m, 2 H), 7.43-7.39 (m, 2 H), 6.78 (t, 1 H); ES-MS *m*/*z* 234.0 [M+H]⁺, LCMS RT (min) 2.32.

### Intermediate F

### Preparation of 1-(4-fluorophenyl)-2-oxo-1,2-dihydropyridine-3-carbonyl chloride

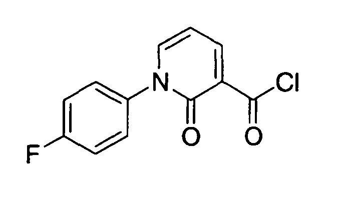

To a mixture 1-(4-fluorophenyl)-2-oxo-1,2-dihydropyridine-3-carboxylic acid (**Intermediate E,** 1.0 g, 4.3 mmol) in thionyl chloride (10 mL) was added DMF (0.1 mL). The reaction mixture was heated at reflux for 4 h, then cooled to rt and concentrated under reduced pressure. The residue was then dissolved in toluene and concentrated under reduced pressure (performed twice). The residue was then dried under mechanical vacuum 1.1 g (100%) of the title compound. ¹H NMR (400 MHz, CD₃OD) δ 8.47 (dd, 1H), 8.21 (dd, 1 H), 7.62-7.59 (m, 2H), 7.43-7.39 (m, 2H), 6.77 (t, 1H).

### Example 1

### Preparation of N-{4-[(6,7-dimethoxyquinolin-4-yl)oxy]phenyl}-1-(4-fluorophenyl)-2-oxo-1,2-dihydropyridine-3-carboxamide

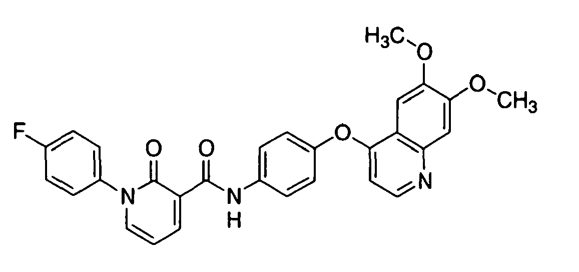

To a solution of 1-(4-fluorophenyl)-2-oxo-1,2-dihydropyridine-3-carboxylic acid (**Intermediate E,** 531 mg, 2.2 mmol) and 4-[(6,7-dimethoxyquinolin-4-yl)oxy]aniline (**Intermediate A,** 450 mg, 1.5 mmol) in DMF (20 mL) were added 1-(3-dimethylaminopropyl)-3-ethylcarbodiimide hydrochloride (437 mg, 2.3 mmol), 1-hydroxybenzotriazole (308 mg, 2.3 mmol), Et₃N (231 mg, 2.27 mmol) and DMAP (18.5 mg, 0.15 mmol). The reaction mixture was stirred at rt for 48 h, and then ethyl acetate (50 mL) was added. The mixture was washed with water, dried (Na₂SO₄), filtered and concentrated under reduced pressure. The residue was triturated with CH₂Cl₂ and filtered to give 275 mg (35%) of the title compound. The filtrate was concentrated under reduced pressure and purified by silica gel flash chromatography to give an additional 180 mg (23 %) of the title compound. Total yield: 455 mg (58%).
¹H NMR (400 MHz, DMSO-*d₆*) δ 12.00 (s, 1 H), 8.50 (dd, 1 H), 8.46 (d, 1 H), 8.11 (dd, 1 H), 7.82 (d, 2 H), 7.62-7.59 (m, 2 H), 7.49 (s, 1 H), 7.43-7.39 (m, 2 H), 7.38 (s, 1 H), 7.25 (d, 2H), 6.71 (t, 1 H), 6.47 (d, 1H), 3.93 (s, 3H), 3.91 (s, 3H); ES-MS *m*/*z* 512.0 [M+H]⁺, LCMS RT (min) 2.61.

### Example 2

### Preparation of N-{4-[(2-aminoquinolin-4-yl)oxy]phenyl}-1-(4-fluorophenyl)-2-oxo-1,2-dihydropyridine-3-carboxamide

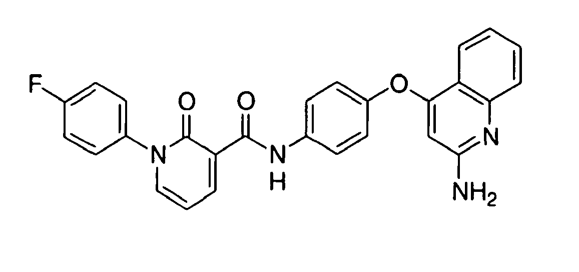

The title compound was synthesized starting from the commercially available 2-amino-4-hydroxy quinoline and using the methods described for **Intermediate A** and **Example 1.**
ES-MS *m*/*z* 467.3 [M+H]⁺, LCMS RT (min) 3.02.

### Example 3

### Preparation of N-{4-[(6,7-dimethoxyquinolin-4-yl)oxy]-3-fluorophenyl}-1-(4-fluorophenyl)-2-oxo-1,2-dihydropyridine-3-carboxamide

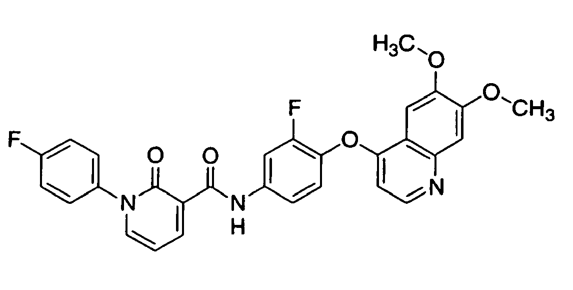

The title compound was synthesized using the methods described for **Example 1** and **Intermediate A** and substituting appropriate starting materials.
ES-MS m/z 530.4 [M+H]⁺, LCMS RT (min) 2.68.

### Example 4

### Preparation of N-{4-[(6,7-dimethoxyquinazolin-4-yl)oxy]phenyl}-1-(4-fluorophenyl)-2-oxo-1,2-dihydropyridine-3-carboxamide

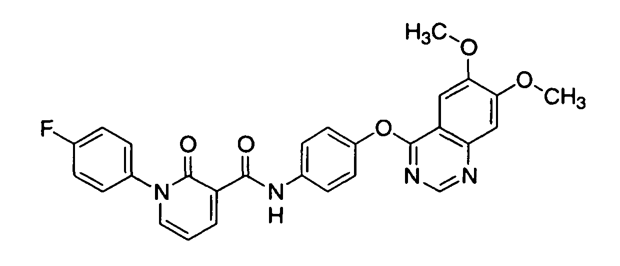

The title compound was synthesized using the methods described for **Example 1** and using **Intermediate C.**
ES-MS *m*/*z* 513.4 [M+H]⁺, LCMS RT (min) 3.11.

### Example 5

### Preparation of N-{4-[(6,7-dimethoxyquinazolin-4-yl)oxy]-2-fluorophenyl}-1-(4-fluorophenyl)-2-oxo-1,2-dihydropyridine-3-carboxamide

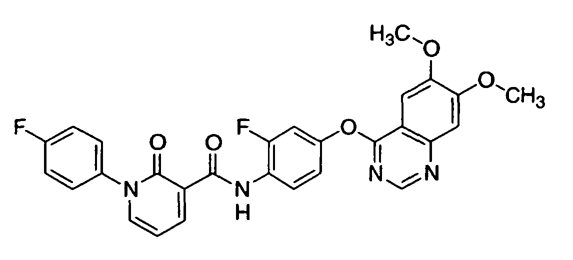

The title compound was synthesized using the methods described for **Example 1** and **Intermediate C,** and substituting for the appropriate starting materials.
ES-MS *m*/*z* 531.3 [M+H]⁺, LCMS RT (min) 3.41.

### Example 6

### Preparation of N-{4-[(6,7-dimethoxyquinolin-4-yl)oxy]-2-fluorophenyl}-1-(4-fluorophenyl)-2-oxo-1,2-dihydropyridine-3-carboxamide

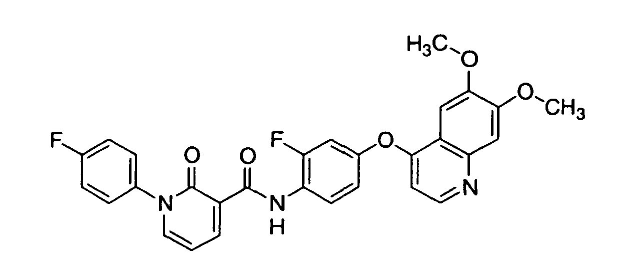

To a solution of 1-(4-fluorophenyl)-2-oxo-1,2-dihydropyridine-3-carbonyl chloride (**Intermediate F,** 52 mg, 0.21 mmol) and 4-[(6,7-dimethoxyquinolin-4-yl)oxyl-2-fluoroaniline (**Intermediate B,** 50 mg, 0.15 mmol) in THF(10 mL) were added Et₃N (81 mg, 0.80 mmol) and DMAP (1.9 mg,0.02 mmol). The reaction mixture was stirred at rt for 20 h and then was concentrated under reduced pressure. The residue was purified by preparative HPLC and the desired fractions were combined and concentrated under reduced pressure. The residue was dissolved in ethyl acetate, and the solution was washed with K₂CO₃ (10% aqueous solution), dried (Na₂SO₄) filtered and concentrated under reduced pressure to give 22 mg (27 %) of the title compound. ¹H NMR (400 MHz, CD₃CN) δ 12.3 (bs, 1 H), 8.68-8.62 (m, 2 H), 8.49 (d, 1 H), 7.82 (dd, 1 H), 7.55 (s, 1 H), 7.53-7.49 (m, 2 H), 7.41 (s, 1 H), 7.36-7.31 (m, 2 H), 7.12 (dd, 1 H), 7.07 (dd, 1 H), 6.66 (t, 1 H), 6.60 (d, 1 H), 4.00 (s, 3 H), 3.96 (s, 3H); ES-MS *m*/*z* 530.3 [M+H]⁺, LCMS RT (min) 2.72.

### Example 7

### Preparation of N-{4-[(6,7-dimethoxyquinazolin-4-yl)thio]-2-fluorophenyl}-1-(4-fluorophenyl)-2-oxo-1,2-dihydropyridine-3-carboxamide

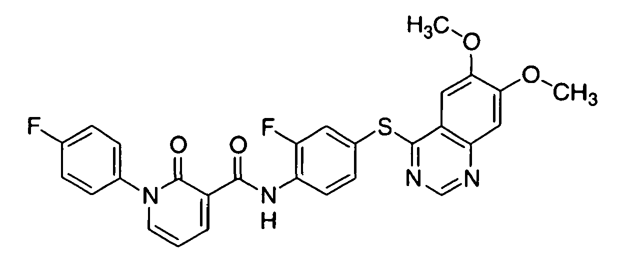

The title compound was synthesized using the methods described for **Example 6** and using **Intermediate D.**
ES-MS *m*/*z* 547.2 [M+H]⁺, LCMS RT (min) 3.66.

### BIOLOGICAL EVALUATION

Demonstration of the activity of the compounds of the present invention may be accomplished through *in vitro*, *ex vivo*, and *in vivo* assays that are well known in the art. For example, to demonstrate the activity of the compounds of the present invention, the following assays may be used.

### Biological Assay Examples

### Flk-1 (murine VEGFR-2) biochemical assay

This assay was performed in 96-well opaque plates (Costar 3915) in the TR-FRET format. Reaction conditions are as follows: 10 µM ATP, 25 nM poly GT-biotin, 2 nM Eu-labelled phospho-Tyr Ab (PY20 Perkin Elmer), 10 nM APC (Perkin Elmer), 7 nM Flk-1 (kinase domain), 1% DMSO, 50 mM HEPES pH 7.5, 10 mM MgCl₂, 0.1 mM EDTA, 0.015% BRIJ, 0.1 mg/mL BSA, 0.1 % β̅mercapto-ethanol). Reaction is initiated upon addition of enzyme. Final reaction volume in each well is 100 µL. Plates are read at both 615 and 665 nM on a Perkin Elmer Victor V Multilabel counter at about 1.5- 2.0 hours after reaction initiation. Signal is calculated as a ratio: (665 nm / 615 nm) * 10000 for each well.

### c-MET Biochemical Assay

An ELISA format is used for the cMET biochemical assay. This assay uses the C-terminal HIS-tagged intracellular kinase domain (956 to 1390 amino acids) human recombinant c-Met in 96-well plates. 96-well plates (Costar # 9018) coated with poly(GluTyr) (Sigma # P0275) are used in this assay. The poly(GluTyr) substrate coated on the plate is phosphorylated in a 100 µL reaction volume with 2 nM cMET protein in an assay buffer (50mM HEPES pH7.0, 5 mM MnCl₂, 0.1 % BSA, 0.5 mM sodium orthovanadate, 0.1% β-mercaptoethanol), with 0.2 µM ATP (Sigma #A7699). 2 µL of compounds are added in as an 8-point IC₅₀ dose curve ranging from 10uM to 128 pM at a final concentration of 1% DMSO. After 25 minutes of incubation, the assay reaction is stopped with 25 µL of 100mM EDTA.The plates are then washed, and wells are treated with 100 µL of 80 ng/mL anti-4G10-HRP antibody (Upstate #16-105) for 1 h. Plates are washed one final time, and are developed with 100 µL 3,3',5,5'-TMB (Sigma #T8665), and quenched with 100 µL 1 M HCl. Plates are read on a Victor 2 plate reader (Perkin Elmer) and IC₅₀ analysis is done using Analyze 5 (in-house software).

### Bcr-Abl wild type and mutant T3151 Biochemical Assay

Abl-T3151 Kinase Filtermat Assay: Inhibition of Abl-T3151 kinase phosphorylation of myelin basic protein by compounds in a ³³P-ATP Filtermat Assay. Mutant Abl-T315I or Abl-wt kinases (0.17 nM) are incubated with Myelin Basic Protein (MBP, 2µM) in assay buffer consisting of 50 mM Tris pH 7.5, 10 mM MgCl₂, 1 mM EGTA, 2 mM DTT, 50 uM ATP and 0.4 µCi of ³³P-ATP. Compounds are added at varying concentrations (final DMSO = 1 %) prior to addition of ATP. The reaction mixture is incubated for 1 hour at 32C. The reaction is then stopped with phosphoric acid (final cone 1 %) and samples are transferred to filtermats and read in a betaplate reader. Inhibition of MBP phosphorylation by Abl-T3151 (or Abl-wt) is analyzed using a 4 parameter fit in Analyze5.

Compounds of this invention showed IC₅₀ < 3 µM in one or more of the biochemical assays discussed above.

### In vitro tumor cell proliferation assay

The adherent tumor cell proliferation assay used to test the compounds of the present invention involves a readout called Cell Titre-Glo developed by Promega (Cunningham, BA "A Growing Issue: Cell Proliferation Assays. Modern kits ease quantification of cell growth" The Scientist 2001, 15(13), 26, and Crouch, SP et al., "The use of ATP bioluminescence as a measure of cell proliferation and cytotoxicity" Journal of Immunological Methods, 1993, 160, 81-88).

H460 cells (lung carcinoma, purchased from ATCC) are plated in 96-well plates at 3000 cells/well in complete media with 10% Fetal Calf Serum and incubated 24 hours at 37°C. Twenty-four hours after plating, test compounds are added over a final concentration range of 10 nM to 20 µM in serial dilutions at a final DMSO concentration of 0.2 %. Cells are incubated for 72 hours at 37°C in complete growth media after addition of the test compound. On day 4, using a Promega Cell Titer Glo Luminescent^{®} assay kit, the cells are lysed and 100 microliters of substrate/buffer mixture is added to each well, mixed and incubated at room temperature for 8 minutes. The samples are read on a luminometer to measure the amount of ATP present in the cell lysates from each well, which corresponds to the number of viable cells in that well. Values read at 24-hour incubation are subtracted as Day 0. For determination of IC₅₀'s, a linear regression analysis can be used to determine drug concentration which results in a 50% inhibition of cell proliferation using this assay format. This protocol was applied to different cell lines of interest, which include, but not limited to, CAKI-1, MNK45, HCC2998, K562, H441, K812, MEG01, SUP15 and HCT116.

The antiproliferative properties of the compounds of this invention can be further characterized using one or more cell lines of interest. Cell lines of interest include, but are not limited to, CAKI-1, MNK45, HCC2998, K562, H441, K812, MEG01, SUP15 and HCT116.

Based on the in vitro test results in which the compounds of the present invention showed IC₅₀ < 3 µM in one or more of the biochemical assays discussed herein above, it is believed that compounds represented by the claimed generic structure would also be active in one or more of the biochemical assayscarried out.

## Claims

1. A compound represented by the formula: 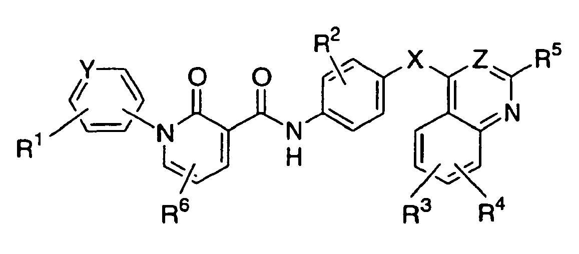 wherein:
X is selected from the group consisting of: O and S;
Y and Z are independently selected from the group consisting of: CH and N;
R¹ is selected from the group consisting of: hydrogen, one or more halogen, NR⁷C(O)R⁸, C(O)OR⁹, OC(O)R¹⁰, OR¹¹, SR¹², cyano, C(O)NR¹⁵R¹⁶, SO₂NR¹⁵R¹⁶, NR¹⁵R¹⁶, linear, branched or cyclic C1-6 alkyl optionally substituted with one or more halogen, OR¹¹, NR¹⁵R¹⁶,
-(CH₂)ᵣNR¹⁵R¹⁶ wherein r is 1, 2 or 3, -O-(CH₂)ₚNR¹⁵R¹⁶, and
-NR¹³-(CH₂)ₚNR¹⁵R¹⁶ wherein p is 2, 3, or 4;
R² is selected from the group consisting of: hydrogen, one or more halogen, OR¹¹, NR¹⁵R¹⁶ and linear, branched or cyclic C1-6 alkyl optionally substituted with halogen, OR¹¹ or NR¹⁵R¹⁶;
R³ and R⁴ are each independently selected from the group consisting of: hydrogen, one or more halogen, NR⁷C(O)R⁸, C(O)OR⁹, OC(O)R¹⁰, OR¹¹, cyano, C(O)NR¹⁵R¹⁶, SO₂NR¹⁵R¹⁶, NR¹⁵R¹⁶, linear, branched or cyclic C1-6 alkyl optionally substituted with halogen, OR¹¹, NR¹⁵R¹⁶, -(CH₂)ᵣNR¹⁵R¹⁶ wherein r is 1, 2 or 3, -O-(CH₂)pNR¹⁵R¹⁶ and
-NR¹³-(CH₂)ₚNR¹⁵R¹⁶ wherein p is 2, 3 or 4;
R⁵ is selected from the group consisting of: hydrogen, OR¹¹, NR¹⁵R¹⁶ linear, branched or cyclic C1-6 alkyl optionally substituted with one or more halogen, and - (CH₂)ᵣNR¹⁵R¹⁶ wherein r is 1, 2 or 3;
R⁶ is selected from the group consisting of: hydrogen, NR⁷C(O)R⁸, C(O)OR⁹, OC(O)R¹⁰, OR¹¹, SR¹², C(O)NR¹⁵R¹⁶, SO₂NR¹⁵R²¹, NR¹⁵R¹⁶, linear, branched or cyclic C1-6 alkyl optionally substituted with one or more halogen, OR⁸, NR¹⁵R¹⁶, - (CH₂)ᵣNR¹⁵R¹⁶ wherein r is 1, 2 or 3,
-O-(CH₂)ₚNR¹⁵R¹⁶ and -NR¹³-(CH₂)ₚNR¹⁵R¹⁶ wherein p is 2, 3 or 4;
R⁷ to R¹⁴ are each independently selected from the group consisting of: hydrogen, linear, branched or cyclic C1-6 alkyl optionally substituted with one or more halogen, OR¹¹, and NR¹⁵R¹⁶;
R¹⁵ and R¹⁶ are each independently selected from the group consisting of: hydrogen, linear, branched or cyclic C1-6 alkyl optionally substituted with one or more halogens. NR¹⁷R¹⁸ and a group wherein R¹⁵ and R¹⁶ together form a five- or six-membered ring optionally containing O or NR¹⁴; and
R¹⁷ and R¹⁸ are each independently selected from the group consisting of: hydrogen, linear, branched or cyclic C1-6 alkyl optionally substituted with one or more halogen, and OR";
pharmaceutically acceptable salts thereof, solvates thereof, hydrates thereof, polymorphs thereof and diastereoisomeric forms thereof including isolated stereoisomers thereof and those within a mixture of stereoisomers.

2. A pharmaceutical composition comprising one or more compounds according to claim 1 and a physiologically acceptable carrier.

3. A composition according to claim 2, further comprising an additional anti-hyper-proliferative agent and/or an additional pharmaceutical agent.

4. A composition according to claim 3, wherein said additional anti-hyper-proliferative agent is selected from the group consisting of: epothiline, a derivative thereof, irinotecan, raloxifen, topotecan and any combination thereof.

5. A composition of claim 3, wherein said additional pharmaceutical agent is selected from the group consisting of: aldesleukin, alendronic acid, alfaferone, alitretinoin, allopurinol, aloprim, aloxi, altretamine, aminoglutethimide, amifostine, amrubicin, amsacrine, anastrozole, anzmet, aranesp, arglabin, arsenic trioxide, aromasin, 5-azacytidine, azathioprine, BCG or tice BCG, bestatin, betamethasone acetate, betamethasone sodium phosphate, bexarotene, bleomycin sulfate, broxuridine, bortezomib, busulfan, calcitonin, campath, capecitabine, carboplatin, casodex, cefesone, celmoleukin, cerubidine, chlorambucil, cisplatin, cladribine, cladribine, clodronic acid, cyclophosphamide, cytarabine, dacarbazine, dactinomycin, DaunoXome, decadron, decadron phosphate, delestrogen, denileukin diftitox, depomedrol, deslorelin, dexrazoxane, diethylstilbestrol, diflucan, docetaxel, doxifluridine, doxorubicin, dronabinol, DW-166HC, eligard, elitek, ellence, emend, epirubicin, epoetin alfa, epogen, eptaplatin, ergamisol, estrace, estradiol, estramustine phosphate sodium, ethinyl estradiol, ethyol, etidronic acid, etopophos, etoposide, fadrozole, farston, filgrastim, finasteride, fligrastim, floxuridine, fluconazole, fludarabine, 5-fluorodeoxyuridine monophosphate, 5-fluorouracil (5-FU), fluoxymesterone, flutamide, formestane, fosteabine, fotemustine, fulvestrant, gammagard, gemcitabine, gemtuzumab, gleevec, gliadel, goserelin, granisetron HCl, histrelin, hycamtin, hydrocortone, eyrthro-hydroxynonyladenine, hydroxyurea, ibritumomab tiuxetan, idarubicin, ifosfamide, interferon alpha, interferon-alpha 2, interferon alfa-2A, interferon alfa-2B, interferon alfa-n1, interferon alfa-n3, interferon beta, interferon gamma-1 a, interleukin-2, intron A, iressa, irinotecan, kytril, lentinan sulphate, letrozole, leucovorin, leuprolide, leuprolide acetate, levamisole, levofolinic acid calcium salt, levothroid, levoxyl, lomustine, lonidamine, marinol, mechlorethamine, mecobalamin, medroxyprogesterone acetate, megestrol acetate, melphalan, menest, 6-mercaptopurine, Mesna, methotrexate, metvix, miltefosine, minocycline, mitomycin C, mitotane, mitoxantrone, Modrenal, Myocet, nedaplatin, neulasta, neumega, neupogen, nilutamide, nolvadex, NSC-631570, OCT-43, octreotide, ondansetron HCl, orapred, oxaliplatin, paclitaxel, pediapred, pegaspargase, Pegasys, pentostatin, picibanil, pilocarpine HCl, pirarubicin, plicamycin, porfimer sodium, prednimustine, prednisolone, prednisone, premarin, procarbazine, procrit, raltitrexed, rebif, rhenium-186 etidronate, rituximab, roferon-A, romurtide, salagen, sandostatin, sargramostim, semustine, sizofiran, sobuzoxane, solu-medrol, sparfosic acid, stem-cell therapy, streptozocin, strontium-89 chloride, sutent, synthroid, tamoxifen, tamsulosin, tasonermin, tastolactone, taxotere, teceleukin, temozolomide, teniposide, testosterone propionate, testred, thioguanine, thiotepa, thyrotropin, tiludronic acid, topotecan, toremifene, tositumomab, trastuzumab, treosulfan, tretinoin, trexall, trimethylmelamine, trimetrexate, triptorelin acetate, triptorelin pamoate, UFT, uridine, valrubicin, vesnarinone, vinblastine, vincristine, vindesine, vinorelbine, virulizin, zinecard, zinostatin stimalamer, zofran, ABI-007, acolbifene, actimmune, affinitak, aminopterin, arzoxifene, asoprisnil, atamestane, atrasentan, sorafenib, avastin, CCI-779, CDC-501, celebrex, cetuximab, crisnatol, cyproterone acetate, decitabine, DN-101, doxorubicin-MTC, dSLIM, dutasteride, edotecarin, eflomithine, exatecan, fenretinide, histamine dihydrochloride, histrelin hydrogel implant, holmium-166 DOTMP, ibandronic acid, interferon gamma, intron-PEG, ixabepilone, keyhole limpet hemocyanin, L-651582, lanreotide, lasofoxifene, libra, lonafarnib, miproxifene, minodronate, MS-209, liposomal MTP-PE, MX-6, nafarelin, nemorubicin, neovastat, nolatrexed, oblimersen, onco-TCS, osidem, paclitaxel polyglutamate, pamidronate disodium, PN-401, QS-21, quazepam, R-1549, raloxifene, ranpirnase, 13-cis -retinoic acid, satraplatin, seocalcitol, T-138067, tarceva, taxoprexin, thymosin alpha 1, tiazofurine, tipifarnib, tirapazamine, TLK-286, toremifene, TransMID-107R, valspodar, vapreotide, vatalanib, verteporfin, vinflunine, Z-100, zoledronic acid and and combinations thereof.

6. A compound of claim 1 selected from the group consisting of compounds represented by the formula: 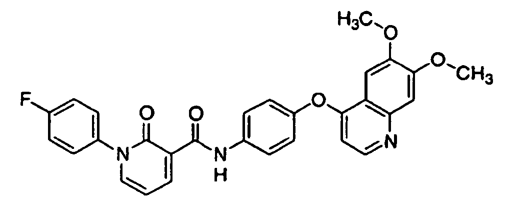 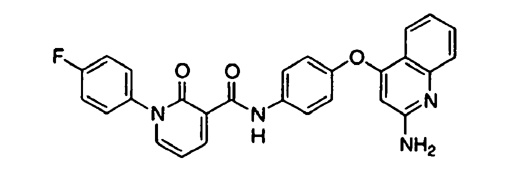 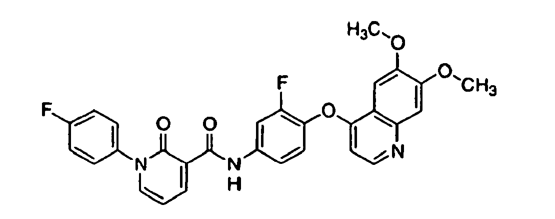 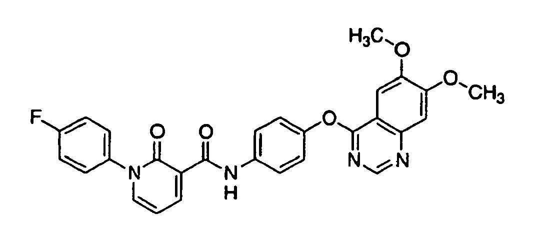 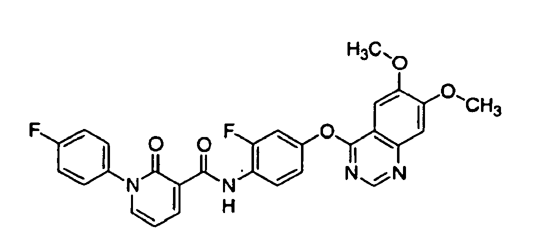 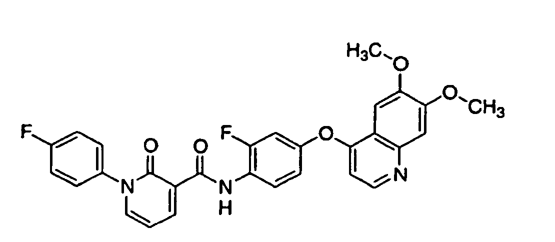 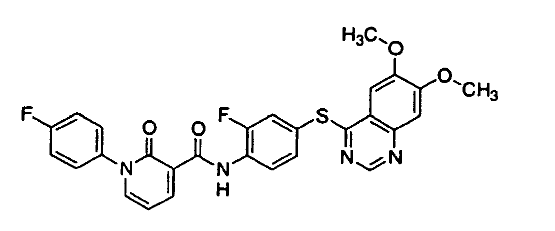 pharmaceutically acceptable salts thereof, solvates thereof, hydrates thereof, polymorphs thereof, diastereoisomeric forms thereof, isolated stereoisomers thereof, and mixtures thereof.

## Patentansprüche

1. Verbindungen der Formel: 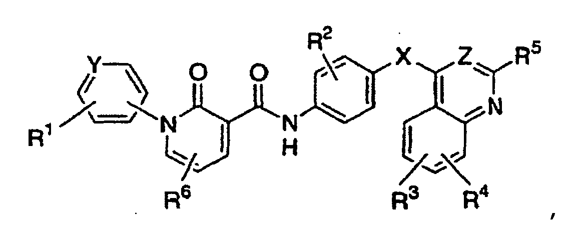 wobei:
X aus der aus O und S bestehenden Gruppe ausgewählt ist;
Y und Z unabhängig voneinander aus der aus CH und N bestehenden Gruppe ausgewählt sind;
R¹ aus der aus Wasserstoff, einem oder mehreren Halogenen, NR⁷C(O)R⁸, C(O)OR⁹, OC(O)R¹⁰, OR¹¹, SR¹², Cyano, C(O)NR¹⁵R¹⁶, SO₂NR¹⁵R¹⁶, NR¹⁵R¹⁶, geradkettigem, verzweigtem oder cyclischem, gegebenenfalls durch ein oder mehrere Halogene substituiertem C₁₋₆-Alkyl, OR¹¹, NR¹⁵R¹⁶,
-(CH₂)ᵣNR¹⁵R¹⁶, wobei r für 1, 2 oder 3 steht, -O-(CH₂)ₚNR¹⁵R¹⁶ und -NR¹³-(CH₂)ₚNR¹⁵R¹⁶, wobei p für 2, 3 oder 4 steht, bestehenden Gruppe ausgewählt ist;
R² aus der aus Wasserstoff, einem oder mehreren Halogenen, OR¹¹, NR¹⁵R¹⁶ und geradkettigem, verzweigtem oder cyclischem, gegebenenfalls durch Halogen substituiertem C₁₋₆-Alkyl, OR¹¹ und NR¹⁵R¹⁶ bestehenden Gruppe ausgewählt ist;
R³ und R⁴ jeweils unabhängig voneinander aus der aus Wasserstoff, einem oder mehreren Halogenen, NR⁷C(O)R⁸, C(O)OR⁹, OC(O)R¹⁰, OR¹¹, Cyano, C(O)NR¹⁵R¹⁶, SO₂NR¹⁵R¹⁶, NR¹⁵R¹⁶, geradkettigem, verzweigtem oder cyclischem, gegebenenfalls durch Halogen substituiertem C₁₋₆-Alkyl, OR¹¹, NR¹⁵R¹⁶, -(CH₂)ᵣNR¹⁵R¹⁶, wobei r für 1, 2 oder 3 steht, -O- (CH₂)ₚNR¹⁵R¹⁶ und
-NR¹³-(CH₂)ₚNR¹⁵R¹⁶, wobei p für 2, 3 oder 4 steht, bestehenden Gruppe ausgewählt sind;
R⁵ aus der aus Wasserstoff, OR¹¹, NR¹⁵R¹⁶, geradkettigem, verzweigtem oder cyclischem, gegebenenfalls durch ein oder mehrere Halogene substituiertem C₁₋₆-Alkyl und -(CH₂)ᵣNR¹⁵R¹⁶, wobei r für 1, 2 oder 3 steht, bestehenden Gruppe ausgewählt ist;
R⁶ aus der aus Wasserstoff, NR⁷C(O)R⁸, C(O)OR⁹, OC(O)R¹⁰, OR¹¹, SR¹², C(O)NR¹⁵R¹⁶, SO₂NR¹⁵R²¹, NR¹⁵R¹⁶, geradkettigem, verzweigtem oder cyclischem, gegebenenfalls durch ein oder mehrere Halogene substituiertem C₁₋₆-Alkyl, OR⁸, NR¹⁵R¹⁶, -(CH₂)ᵣNR¹⁵R¹⁶, wobei r für 1, 2 oder 3 steht, -O-(CH₂)ₚNR¹⁵R¹⁶ und -NR¹³-(CH₂)ₚNR¹⁵R¹⁶, wobei p für 2, 3 oder 4 steht, bestehenden Gruppe ausgewählt ist;
R⁷ bis R¹⁴ jeweils unabhängig voneinander aus der aus Wasserstoff, geradkettigem, verzweigtem oder cyclischem, gegebenenfalls durch ein oder mehrere Halogene substituiertem C₁₋₆-Alkyl, OR¹¹ und NR¹⁵R¹⁶ bestehenden Gruppe ausgewählt sind;
R¹⁵ und R¹⁶ jeweils unabhängig voneinander aus der aus Wasserstoff, geradkettigem, verzweigtem oder cyclischem, gegebenenfalls durch ein oder mehrere Halogene substituiertem C₁₋₆-Alkyl, NR¹⁷R¹⁸ und einer Gruppe, in welcher R¹⁵ und R¹⁶ zusammen einen fünf-oder sechsgliedrigen Ring bilden, der gegebenenfalls O oder NR¹⁴ enthält, bestehenden Gruppe ausgewählt sind; und
R¹⁷ und R¹⁸ jeweils unabhängig voneinander aus der aus Wasserstoff, geradkettigem, verzweigtem oder cyclischem, gegebenenfalls durch ein oder mehrere Halogene substituiertem C₁₋₆-Alkyl und OR¹¹ bestehenden Gruppe ausgewählt sind;
deren pharmazeutisch unbedenkliche Salze, deren Solvate, deren Hydrate, deren Polymorphe und deren diastereoisomere Formen einschließlich isolierter Stereoisomere davon und denen in einer Mischung von Stereoisomeren.

2. Pharmazeutische Zusammensetzung, enthaltend eine oder mehrere Verbindungen nach Anspruch 1 und einen physiologisch unbedenklichen Träger.

3. Zusammensetzung nach Anspruch 2, weiterhin enthaltend ein zusätzliches antihyperproliferatives und/oder ein zusätzliches pharmazeutisches Mittel.

4. Zusammensetzung nach Anspruch 3, wobei das zusätzliche antihyperproliferative Mittel aus der aus Epothilin, einem Derivat davon, Irinotecan, Raloxifen, Topotecan und beliebigen Kombinationen davon bestehenden Gruppe ausgewählt ist.

5. Zusammensetzung nach Anspruch 3, wobei das zusätzliche pharmazeutische Mittel aus der aus Aldesleukin, Alendronsäure, Alfaferon, Alitretinoin, Allopurinol, Aloprim, Aloxi, Altretamin, Aminoglutethimid, Amifostin, Amrubicin, Amsacrin, Anastrozol, Anzmet, Aranesp, Arglabin, Arsentrioxid, Aromasin, 5-Azacytidin, Azathioprin, BCG oder tice-BCG, Bestatin, Betamethason-Acetat, Betamethason-Natriumphosphat, Bexaroten, Bleomycin-Sulfat, Broxuridin, Bortezomib, Busulfan, Calcitonin, Campath, Capecitabin, Carboplatin, Casodex, Cefeson, Celmoleukin, Cerubidin, Chlorambucil, Cisplatin, Cladribin, Clodronsäure, Cyclophosphamid, Cytarabin, Dacarbazin, Dactinomycin, DaunoXome, Decadron, Decadron-Phosphat, Delestrogen, Denileukin Diftitox, Depomedrol, Deslorelin, Dexrazoxan, Diethylstilbestrol, Diflucan, Docetaxel, Doxifluridin, Doxorubicin, Dronabinol, DW-166HC, Eligard, Elitek, Ellence, Emend, Epirubicin, Epoetin-alfa, Epogen, Eptaplatin, Ergamisol, Estrace, Estradiol, Estramustin-Natriumphosphat, Ethinylestradiol, Ethyol, Etidronsäure, Etopophos, Etoposid, Fadrozol, Farston, Filgrastim, Finasterid, Fligrastim, Floxuridin, Fluconazol, Fludarabin, 5-Fluordeoxyuridin-Monophosphat, 5-Fluoruracil (5-FU), Fluoxymesteron, Flutamid, Formestan, Fosteabin, Fotemustin, Fulvestrant, Gammagard, Gemcitabin, Gemtuzumab, Gleevec, Gliadel, Goserelin, Granisetron-HCl, Histrelin, Hycamtin, Hydrocorton, erythro-Hydroxynonyladenin, Hydroxyharnstoff, Ibritumomab Tiuxetan, Idarubicin, Ifosfamid, Interferon-alpha, Interferon-alpha-2, Interferon-alpha-2α, Interferon-alpha-2β, Interferon-alpha-n1, Interferon-alpha-n3, Interferon-beta, Interferon-gamma-1α, Interleukin-2, Intron A, Iressa, Irinotecan, Kytril, Lentinan-Sulfat, Letrozol, Leucovorin, Leuprolid, Leuprolid-Acetat, Levamisol, Levofolinsäure-Calciumsalz, Levothroid, Levoxyl, Lomustin, Lonidamin, Marinol, Mechlorethamin, Mecobalamin, Medroxyprogesteron-Acetat, Megestrol-Acetat, Melphalan, Menest, 6-Mercaptopurin, Mesna, Methotrexat, Metvix, Miltefosin, Minocyclin, Mitomycin C, Mitotan, Mitoxantron, Modrenal, Myocet, Nedaplatin, Neulasta, Neumega, Neupogen, Nilutamid, Nolvadex, NSC-631570, OCT-43, Octreotid, Ondansetron-HCl, Orapred, Oxaliplatin, Paclitaxel, Pediapred, Pegaspargase, Pegasys, Pentostatin, Picibanil, Pilocarpin-HCl, Pirarubicin, Plicamycin, Porfimer-Natrium, Prednimustin, Prednisolon, Prednison, Premarin, Procarbazin, Procrit, Raltitrexed, Rebif, Rhenium-186-Etidronat, Rituximab, Roferon-A, Romurtid, Salagen, Sandostatin, Sargramostim, Semustin, Sizofiran, Sobuzoxan, Solu-Medrol, Sparfosinsäure, Stammzellentherapie, Streptozocin, Strontium-89-chlorid, Sutent, Synthroid, Tamoxifen, Tamsulosin, Tasonermin, Tastolacton, Taxoter, Teceleukin, Temozolomid, Teniposid, Testosteron-Propionat, Testred, Thioguanin, Thiotepa, Thyrotropin, Tiludronsäure, Topotecan, Toremifen, Tositumomab, Trastuzumab, Treosulfan, Tretinoin, Trexall, Trimethylmelamin, Trimetrexat, Triptorelin-Acetat, Triptorelin-Pamoat, UFT, Uridin, Valrubicin, Vesnarinon, Vinblastin, Vincristin, Vindesin, Vinorelbin, Virulizin, Zinecard, Zinostatin-Stimalamer, Zofran, ABI-007, Acolbifen, Actimmun, Affinitak, Aminopterin, Arzoxifen, Asoprisnil, Atamestan, Atrasentan, Sorafenib, Avastin, CCI-779, CDC-501, Celebrex, Cetuximab, Crisnatol, Cyproteron-Acetat, Decitabin, DN-101, Doxorubicin-MTC, dSLIM, Dutasterid, Edotecarin, Eflornithin, Exatecan, Fenretinid, Histamin-Dihydrochlorid, Histrelin-Hydrogel-Implant, Holmium-166-DOTMP, Ibandronsäure, Interferon-gamma, Intron-PEG, Ixabepilon, Keyhole Limpet-Hemocyanin, L-651582, Lanreotid, Lasofoxifen, Libra, Lonafarnib, Miproxifen, Minodronat, MS-209, liposomales MTP-PE, MX-6, Nafarelin, Nemorubicin, Neovastat, Nolatrexed, Oblimersen, Onko-TCS, Osidem, Paclitaxel-Polyglutamat, Pamidronat-Dinatrium, PN-401, QS-21, Quazepam, R-1549, Raloxifen, Ranpirnase, 13-*cis-*Retinsäure, Satraplatin, Seocalcitol, T-138067, Tarceva, Taxoprexin, Thymosin-alpha-1, Tiazofurin, Tipifarnib, Tirapazamin, TLK-286, Toremifen, TransMID-107R, Valspodar, Vapreotid, Vatalanib, Verteporfin, Vinflunin, Z-100, Zoledronsäure, sowie Kombinationen davon bestehenden Gruppe ausgewählt ist.

6. Verbindungen nach Anspruch 1, ausgewählt aus der Gruppe von Verbindungen, die durch die folgenden Formeln wiedergegeben werden: 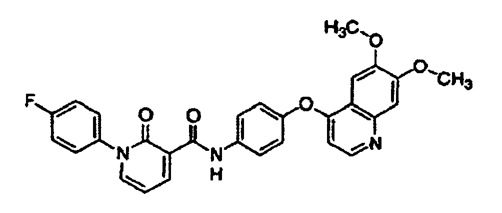 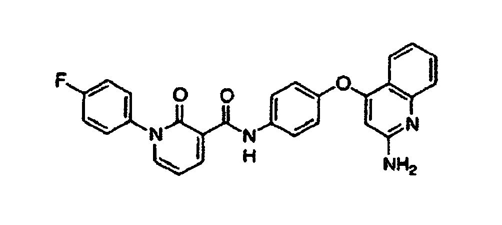 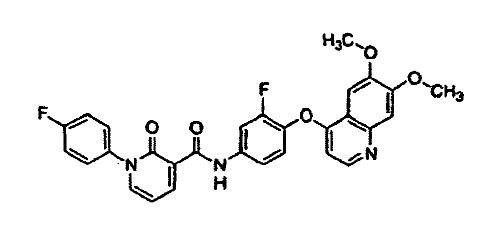 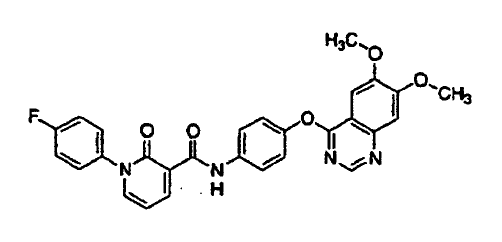 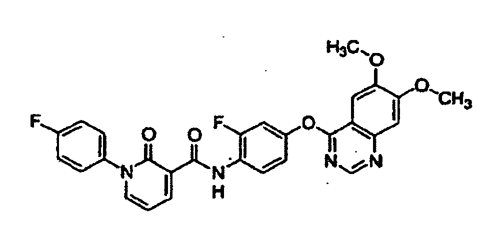 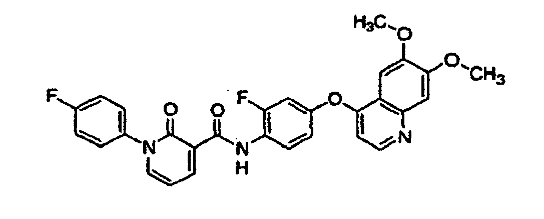 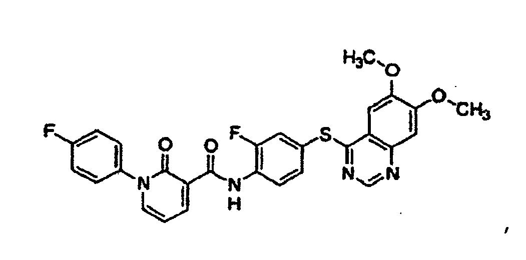 deren pharmazeutisch unbedenkliche Salze, deren Solvate, deren Hydrate, deren Polymorphe, deren diastereoisomere Formen, deren isolierte Stereoisomere und Mischungen davon.

## Revendications

1. Composé de formule : 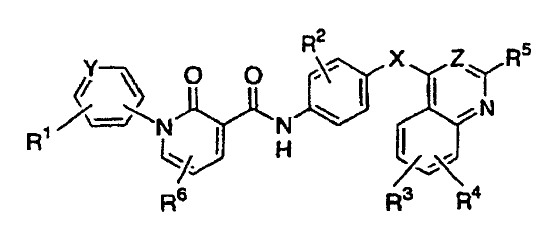 où :
X est choisi dans le groupe constitué par : O et S ;
Y et Z sont indépendamment choisis dans le groupe constitué par : CH et N ;
R¹ est choisi dans le groupe constitué par les atomes et groupements suivants : hydrogène, un ou plusieurs halogènes, NR⁷C(O)R⁸, C(O)OR⁹, OC(O)R¹⁰, OR¹¹, SR¹², cyano, C(O)NR¹⁵R¹⁶, SO₂NR¹⁵R¹⁶, NR¹⁵R¹⁶, alkyle linéaire, ramifié ou cyclique en C1-6 éventuellement substitué par un ou plusieurs atomes d'halogène, OR¹¹, NR¹⁵R¹⁶, -(CH₂)ᵣR¹⁵R¹⁶ où r est égal à 1, 2 ou 3, -(O-CH₂)ₚNR¹⁵R¹⁶, et
-NR¹³-(CH₂)ₚNR¹⁵R¹⁶ où p est égal à 2, 3, ou 4 ;
R² est choisi dans le groupe constitué par les atomes et groupements suivants : hydrogène, un ou plusieurs halogènes, OR¹¹, NR¹⁵R¹⁶ et alkyle linéaire, ramifié ou cyclique en C1-6 éventuellement substitué par un ou plusieurs atomes d'halogène, OR¹¹ ou NR¹⁵R¹⁶;
chacun des radicaux R³ et R⁴ est indépendamment choisi dans le groupe constitué par les atomes et groupements suivants : hydrogène, un ou plusieurs halogènes, NR⁷C(O)R⁸, C(O)OR⁹, OC(O)R¹⁰, OR¹¹, cyano, C(O)NR¹⁵R¹⁶, SO₂NR¹⁵R¹⁶, NR¹⁵R¹⁶, alkyle linéaire, ramifié ou cyclique en C1-6 éventuellement substitué par un ou plusieurs atomes d'halogène, OR¹¹, NR¹⁵R¹⁶, -(CH2)ᵣNR¹⁵R¹⁶ où r est égal à 1, 2 ou 3, -O-(CH₂)ₚNR¹⁵R¹⁶ et
-NR¹³-(CH₂)ₚNR¹⁵R¹⁶ où p est égal à 2, 3 ou 4 ;
R⁵ est choisi dans le groupe constitué par les atomes et groupements suivants : hydrogène, OR¹¹, NR¹⁵R¹⁶, alkyle linéaire, ramifié ou cyclique en C1-6 éventuellement substitué par un ou plusieurs halogènes, et - (CH₂)ᵣNR¹⁵R¹⁶ où r est égal à 1, 2 ou 3 ;
R⁶ est choisi dans le groupe constitué par les atomes et groupements suivants : hydrogène, NR⁷C(O)R⁸, C(O)OR⁹, OC(O)R¹⁰, OR¹¹, SR¹², C(O)NR¹⁵R¹⁵, SO₂NR¹⁵R²¹, NR¹⁵R¹⁶, alkyle linéaire, ramifié ou cyclique en C1-6 éventuellement substitué par un ou plusieurs atomes d'halogène, OR⁸, NR¹⁵R¹⁶, -(CH2)ᵣNR¹⁵R¹⁶ où r est égal à 1, 2 ou 3,
-O-(CH₂)ₚNR¹⁵R¹⁶ et -NR¹³-(CH₂)pNR¹⁵R¹⁶ où p est égal à 2, 3 ou 4 ;
chacun des radicaux R⁷ à R¹⁴ est indépendamment choisi dans le groupe constitué par les atomes et groupements suivants : hydrogène, alkyle linéaire, ramifié ou cyclique en C1-6 éventuellement substitué par un ou plusieurs halogènes, OR¹¹ et NR¹⁵R¹⁶;
chacun des radicaux R¹⁵ et R¹⁶ est indépendamment choisi dans le groupe constitué par les atomes et groupements suivants : hydrogène, alkyle linéaire, ramifié ou cyclique en C1-6 éventuellement substitué par un ou plusieurs halogènes, NR¹⁷R¹⁸ et un groupement où R¹⁵ et R¹⁶ forment ensemble un cycle à cinq ou six chaînons incluant éventuellement O ou NR¹⁴; et
chacun des radicaux R¹⁷ et R¹⁸ est indépendamment choisi dans le groupe constitué par les atomes et groupements suivants : hydrogène, alkyle linéaire, ramifié ou cyclique en C1-6 éventuellement substitué par un ou plusieurs halogènes, et OR¹¹ *;*
leurs sels de qualité pharmaceutique, leurs solvates, leurs hydrates, leurs formes polymorphiques et leurs formes diastéréoisomères, y compris leurs stéréoisomères isolés et ceux qui sont inclus dans un mélange de stéréoisomères.

2. Composition pharmaceutique comprenant un ou plusieurs composés conformes à la revendication 1 et un vecteur ou un diluant de qualité physiologique.

3. Composition conforme à la revendication 2, comprenant en outre un agent anti-hyperprolifératif supplémentaire et/ou un agent pharmaceutique supplémentaire.

4. Composition conforme à la revendication 3, où ledit agent anti-hyperprolifératif supplémentaire est choisi dans le groupe constitué par les suivantes : épothiline, l'un de ses dérivés, irinotécan, raloxifène, topotécan et n'importe laquelle de leurs combinaisons.

5. Composition conforme à la revendication 3, où ou ledit agent pharmaceutique supplémentaire est choisi dans le groupe constitué par les suivantes : aldesleukine, acide alendronique, alfaférone, alitrétinoïne, allopurinol, aloprim, aloxi, altrétamine, aminoglutéthimide, amifostine, amrubicine, amsacrine, anastrozole, anzmet, aranesp, arglabine, trioxyde d'arsenic, aromasine, 5-azacytidine, azathioprine, BCG ou Tice BCG, bestatine, bétaméthasone acétate, betaméthasone sodium phosphate, bexarotène, bléomycine sulfate, broxuridine, bortézomib, busulfan, calcitonine, campath, capécitabine, carboplatine, casodex, céfésone, celmoleukine, cérubidine, chlorambucil, cisplatine, cladribine, cladribine, acide clodronique, cyclophosphamide, cytarabine, dacarbazine, dactinomycine, Daunoxome, décadron, décadron phosphate, délestrogène, déniteukine diftitox, dépomédrol, desloréline, dexrazoxane, diéthylstilbéstrol, diflucan, docétaxel, doxifluridine, doxorubicine, dronabinol, DW-166HC, éligard, élitek, ellence, emend, épirubicine, époïétine alfa, épogène, eptaplatine, ergamisol, estrace, oestradiol, oestramustine phosphate sodium, éthinyl-oestradiol, éthyol, acide étidronique, étopophos, étoposide, fadrozole, farston, filgrastim, finastéride, fligrastim, floxuridine, fluconazole, fludarabine, 5-fluorodésoxyuridine monophosphate, 5-fluorouracile (5-FU), fluoxymestérone, flutamide, formestane, fostéabine, fotémustine, fulvéstrant, gammagard, gemcitabine, gemtuzumab, glivec, gliadel, goséréline, granisétron HCl, histréline, hycamtine, hydrocortone, érythro-hydroxynonyladénine, hydroxyurée, ibritumomab tiuxétan, idarubicine, ifosfamide, interféron alpha, interféron-alpha 2, interféron alfa-2A, interféron alfa-2B, interféron alfa-n1, interféron alfa-n3, interféron bêta, interféron gamma-1a, interleukine-2, intron A, iressa, irinotécan, kytril, lentinan sulphate, létrozole, leucovorine, leuprolide, leuprolide acétate, lévamisole, acide lévofolinique-sel de calcium, lévothroïde, lévoxyl, lomustine, lonidamine, marinol, méchloréthamine, mécobalamine, médroxyprogestérone acétate, mégéstrol acétate, melphalan, ménest, 6-mercaptopurine, Mesna, méthotrexate, metvix, miltéfosine, minocycline, mitomycine C, mitotane, mitoxantrone, Modrénal, Myocet, nédaplatine, neulasta, neuméga, neupogène, nilutamide, nolvadex, NSC-631570, OCT-43, octréotide, ondansétron HCl, orapred, oxaliplatine, paclitaxel, pédiapred, pégaspargase, Pegasys, pentostatine, picibanil, pilocarpine HCl, pirarubicine, plicamycine, porfimer sodium, prednimustine, prednisolone, prednisone, prémarine, procarbazine, procrit, raltitrexed, rebif, rhénium-186 étidronate, rituximab, roféron-A, romurtide, salagène, sandostatine, sargramostim, sémustine, sizofiran, sobuzoxane, solumédrol, acide sparfosique, thérapie par cellules souches, streptozocine, chlorure de strontium-89, sutent, synthroïde, tamoxifène, tamsulosine, tasonermine, tastolactone, taxotère, técéleukine, témozolomide, téniposide, testostérone propionate, testred, thioguanine, thiotépa, thyrotropine, acide tiludronique, topotécan, torémifène, tositumomab, trastuzumab, tréosulfan, trétinoïne, trexall, triméthylmélamine, trimétrexate, triptoréline acétate, triptoréline pamoate, UFT, uridine, valrubicine, vesnarinone, vinblastine, vincristine, vindésine, vinorelbine, virulizine, zinécard, zinostatine stimalamère, zofran, ABI-007, acolbifène, actimmune, affinitak, aminopterine, arzoxifène, asoprisnil, atamestane, atrasentan, sorafénib, avastine, CCI-779, CDC-501, célébrex, cétuximab, crisnatol, cyprotérone acétate, décitabine, DN-101, doxorubicine-MTC, dSLIM, dutastéride, édotécarine, éflomithine, exatécan, fenrétinide, histamine dichlorhydrate, histréline-implant d'hydrogel, holmium-166 DOTMP, acide ibandronique, interféron gamma, intron-PEG, ixabépilone, hémocyanine de fissurelle, L-651582, lanréotide, lasofoxifène, librax, lonafarnib, miproxifène, minodronate, MS-209, MTP-PE liposomial, MX-6, nafaréline, némorubicine, néovastat, nolatrexed, oblimersène, onco-TCS, osidem, paclitaxel polyglutamate, pamidronate disodium, PN-401, QS-21, quazépam, R-1549, raloxifène, ranpimase, acide 13-cis-rétinoïque, satraplatine, séocalcitol, T-138067, tarcéva, taxoprexine, thymosine alpha 1, tiazofurine, tipifamib, tirapazamine, TLK-286, torémifène, TransMID-107R, valspodar, vapréotide, vatalanib, vertéporfine, vinflunine, Z-100, acide zolédronique et leurs combinaisons.

6. Composé conforme à la revendication 1, choisi dans le groupe constitué par les composés de formule suivants : 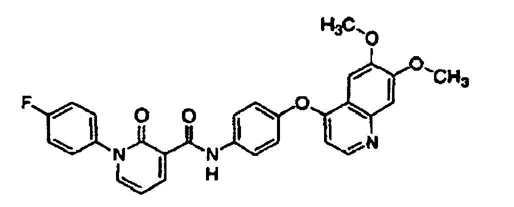 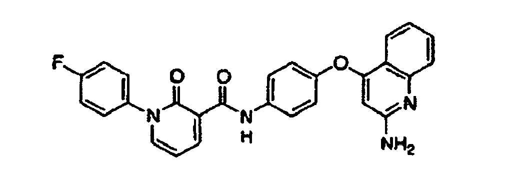 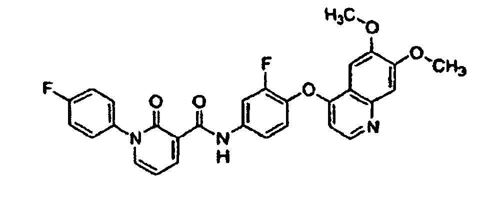 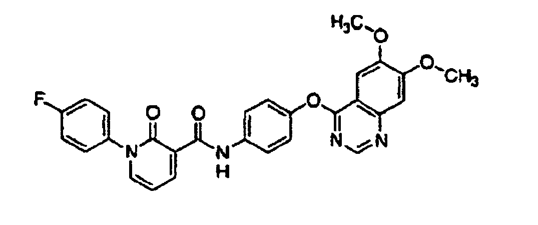 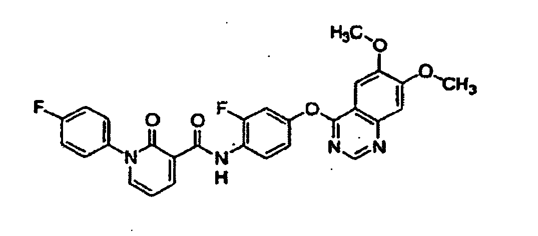 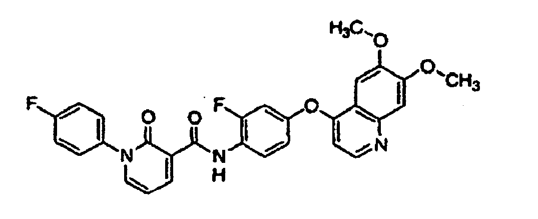 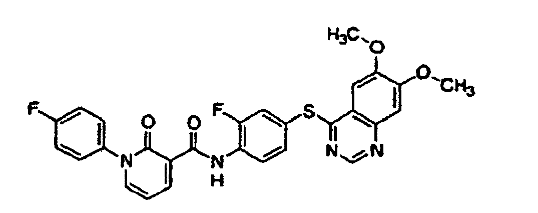 leurs sels de qualité pharmaceutique, leurs solvates, leurs hydrates, leurs formes polymorphiques, leurs formes diastéréoisomères, leurs stéréoisomères isolés et leurs mélanges.
